# EUROPEAN PATENT APPLICATION

(11) **EP 4 703 382 A1**
(43) Date of publication of application: **04.03.2026**
(21) Application number: 24792158.8
(22) Date of filing: 19.04.2024
(51) Int. Cl.: C07K 16/28, A61P 37/02, C07K 16/46, A61P 35/00, A61K 39/395, C12N 15/13

(54) **ANTI-PD-L1 NANOBODY, PREPARATION METHOD THEREFOR AND USE THEREOF**

(30) Priority: 20.04.2023 CN 202310427416
(71) Applicant: Kanglitai Biomedical (Qingdao) Co., Ltd., Qingdao, Shandong 266111 (CN)
(72) Inventor: XU, Yinfeng, Qingdao, Shandong 266111 (CN); ZHENG, Binbin, Qingdao, Shandong 266111 (CN); WANG, Lu, Qingdao, Shandong 266111 (CN); LI, Lei, Qingdao, Shandong 266111 (CN); LI, Tingting, Qingdao, Shandong 266111 (CN); TANG, Tian, Qingdao, Shandong 266111 (CN); JIANG, Lizhong, Qingdao, Shandong 266111 (CN); WANG, Jiangang, Qingdao, Shandong 266111 (CN); WU, Mingyuan, Qingdao, Shandong 266111 (CN)
(74) Representative: Michalski Hüttermann & Partner Patentanwälte mbB
(86) International application number: PCT/CN2024/088960
(87) International publication number: WO 2024/217575

(57) **Abstract**

An anti-PD-L1 nanobody, a preparation method therefor and the use thereof. The anti-PD-L1 nanobody is prepared by means of screening. The anti-PD-L1 nanobody has high affinity and specificity, can efficiently target PD-L1, has a simple structure, is easy to prepare, and can be further used for preparing PD-L1-binding molecules, immunoconjugates, etc. Therefore, the anti-PD-L1 nanobody has wide application prospects in the fields of immunodetection, tumor treatment, etc.

## Description

The present application claims priority to CN 202310427416.0 (filed on April 20, 2023, with the invention title "An Anti-PD-L1 Nanobody, Preparation Method and Use Thereof").

### Technical field

The present invention belongs to the field of biotechnology and relates to an anti-PD-L1 nanobody, and preparation methods and uses thereof.

### Background

Programmed cell death 1 ligand 1 (PD-L1), also known as cluster of differentiation 274 (CD274) or B7 homolog 1 (B7-H1), is a protein in humans. It is encoded by the CD274 gene and is a type I transmembrane protein with a molecular weight of 40 kD, belonging to the B7 family.

It was studied that PD-L1 protein is primarily expressed on antigen-presenting cells (APCs), activated T cells, B cells, macrophages, placental trophoblast cells, cardiac endothelial cells, and cortical thymic epithelial cells. Elevated expression of PD-L1 has been detected in various types of human tumor tissues, with levels generally higher than those in normal tissues. When the T cell surface receptor PD-1 binds to its ligand PD-L1, it recruits the protein tyrosine phosphatases SHP-1 (src homology region 2 domain-containing phosphatase-1) and SHP-2, generating an inhibitory signal that suppresses the phosphorylation of the downstream effector PI3K/Akt pathway and the activation of mTOR and ERK2, thereby promoting the differentiation of CD4⁺ Foxp3⁻ T cells into CD4⁺ Foxp3⁺ regulatory Tregs. In short, increased expression of PD-L1 on the surface of tumor cells within the tumor microenvironment, when binding to PD-1 on activated T cells, transmits a negative regulatory signal that leads to the apoptosis or immune anergy of tumor antigen-specific T cells, thereby suppressing the immune response. It has been discovered that using antibodies to block the PD-1/PD-L1 signaling pathway can restore T cells' ability to kill tumor cells, helping cancer patients eliminate cancer cells through their own immune responses. Antibodies targeting the PD-1/PD-L1 signaling pathway have broad application prospects in the field of tumor therapy.

Nanobodies were first reported in 1993 by Belgian scientists in "Nature", which are a type of antibodies naturally lacking a light chain found in the peripheral blood of alpacas. This antibody comprises only one heavy chain variable region (VHH) and two conventional CH2 and CH3 domains. The VHH structure individually cloned and expressed, retains structural stability and antigen-binding activity, comparable to that of the original heavy-chain antibody. thereby making it the smallest known unit capable of binding to target antigens. The VHH crystal measures 2.5 nm in width and 4 nm in length, with a molecular weight of only 15 kDa; therefore, it is also named as a nanobody (Nb). Nanobodies possess several advantages: small molecular weight, which enables easy penetration into dense tissues and even the blood-brain barrier; high affinity, strong specificity, good solubility, and good stability; weak immunogenicity in humans and excellent biocompatibility; high expression in prokaryotic or eukaryotic systems, facilitating production; and simple structure, which is readily to be engineered.

In summary, it is of significant importance to develop novel nanobodies targeting PD-L1 in the field of cancer treatment.

### Summary of the Invention

Regarding the shortcomings in the prior art and practical needs, the present invention provides an anti-PD-L1 nanobody, along with preparation methods and uses thereof. In the present invention, an anti-PD-L1 nanobody with high affinity was screened and prepared, demonstrating a promising application prospect in fields, such as immunological detection and tumor therapy.

To achieve the above purpose, following technical solutions were used in the present invention:
In the first aspect, an anti-PD-L1 nanobody is provided in the present invention, wherein the complementarity-determining regions (CDRs) of the VHH chain of the anti-PD-L1 nanobody are selected from any one of following groups:
(1) CDR1, CDR2, and CDR3 with amino acid sequences respectively shown in SEQ ID NO: 1, SEQ ID NO: 2, and SEQ ID NO: 3;
(2) CDR1, CDR2, and CDR3 with amino acid sequences respectively shown in SEQ ID NO: 4, SEQ ID NO: 5, and SEQ ID NO: 6;
(3) CDR1, CDR2, and CDR3 with amino acid sequences respectively shown in SEQ ID NO: 7, SEQ ID NO: 8, and SEQ ID NO: 9;
(4) CDR1, CDR2, and CDR3 with amino acid sequences respectively shown in SEQ ID NO: 10, SEQ ID NO: 11, and SEQ ID NO: 12.

In the present invention, anti-PD-L1 nanobodies are screened. The anti-PD-L1 nanobodies only comprise a heavy chain variable region with high affinity and specificity, are capable of highly efficiently targeting PD-L1, have simple structures, are readily to be prepared, and exhibit promising application prospects in fields such as immunological detection and tumor therapy.
SEQ ID NO: 1: GFTVSNSAVS.
SEQ ID NO: 2: LIDSNRNTV.
SEQ ID NO: 3: KGIWGSTL.
SEQ ID NO: 4: GSIFRITPVA.
SEQ ID NO: 5: VITDNGSTD.
SEQ ID NO: 6: SRARPVRWT.
SEQ ID NO: 7: GFTFSTSFVS.
SEQ ID NO: 8: RISPGGASTS.
SEQ ID NO: 9: REIYSGRFSGFDY.
SEQ ID NO: 10: GIISDINMVA.
SEQ ID NO: 11: KIFRGGSTY.
SEQ ID NO: 12: ARIQIRADRDYSDY.

It can be understood that, in another aspect, the present invention claims the heavy chain variable region VHH of a nanobody, wherein the complementarity-determining regions (CDRs) of the VHH are selected from any one of the following groups:
(1) CDR1, CDR2, and CDR3 with amino acid sequences respectively shown in SEQ ID NO: 1, SEQ ID NO: 2, and SEQ ID NO: 3;
(2) CDR1, CDR2, and CDR3 with amino acid sequences respectively shown in SEQ ID NO: 4, SEQ ID NO: 5, and SEQ ID NO: 6;
(3) CDR1, CDR2, and CDR3 with amino acid sequences respectively shown in SEQ ID NO: 7, SEQ ID NO: 8, and SEQ ID NO: 9;
(4) CDR1, CDR2, and CDR3 with amino acid sequences respectively shown in SEQ ID NO: 10, SEQ ID NO: 11, and SEQ ID NO: 12.

It is to be understood that any derivative sequence, which is generated by optionally adding, deleting, modifying, and/or substituting at least one (e.g., 1-3, preferably 1-2, more preferably 1) amino acid based on any one of the above amino acid sequences, and retains the ability to bind to PD-L1, shall fall within the protection scope of the present invention.

In a specific embodiment of the present invention, the derivative sequence, which has undergone addition, deletion, modification, and/or substitution of at least one amino acid and is capable of retaining the ability to specifically bind to PD-L1, exhibits a homology or sequence identity of at least 88%, at least 89%, at least 90%, at least 91%, at least 92%, at least 93%, at least 94%, at least 95%, at least 96%, at least 97%, at least 98%, or at least 99% in amino acid sequence.

Preferably, the amino acid sequence of the VHH chain of the anti-PD-L1 nanobody is selected from any one of the sequences shown in SEQ ID NO: 13 to SEQ ID NO: 16 or a sequence having at least 80% homology thereto, including but not limited to amino acid sequences with at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% homology.
SEQ ID NO:13: 
QVQLQESGGGLVQPGGSLTLSCAASGFTVSNSAVSWARQAPGKGLEWVSLIDSNRN
TVYAQSVRGRFTISRDNVKNTVYLQMNSLKPEDTAVYYCAKGIWGSTLTGQGTQVTVSS.
SEQ ID NO:14: 
QVQLQESGGGLVQPGGSLRLSCAGSGSIFRITPVAWYRQAPEKQRELVAVITDNGST
DYGDFVKGRFTISRDNAKNTVYLQMNSLKPEDTAVYYCNSRARPVRWTWGQGTQVTVS
S.
SEQ ID NO:15: 
QVQLQESGGGLVQPGGSLRLSCAASGFTFSTSFVSWVRQAPGKGLEWVSRISPGGAS
TSYADSVKGRFTISRDNAENTLYLQMNSLKPEDTALYYCAREIYSGRFSGFDYWGQGTQV
TVSS.
SEQ ID NO:16:

QVQLQESGGGLVQPGGSLRLSCAASGIISDINMVAWYRQAPGKQRELVAKIFRGGST
YYTDFVKGRFTISRENAKNTVYLQMNSLKPDDTAVYYCNARIQIRADRDYSDYWGQGTQ
VTVSS.

In the second aspect, a PD-L1 binding molecule is provided in the present invention, comprising the anti-PD-L1 nanobody or the heavy chain variable region VHH of the first aspect.

In the present invention, PD-L1 heavy chain single-domain antibodies (nanobodies) with high affinity and specificity are screened and prepared. Based on these antibodies, various different PD-L1-binding molecules can be prepared, such as those conjugated with optional tag sequences for facilitating expression and/or purification; humanized antibodies obtained through humanization; or prepared multispecific antibodies, including bivalent antibodies and/or polyvalent antibodies. The multispecific antibodies comprise one or more anti-PD-L1 nanobodies and/or VHH domains of the first aspect, and may further comprise antibodies targeting any other antigen.

In a specific embodiment, the tag sequence comprises any one or a combination of at least two from an Fc tag, an HA tag, a GGGS sequence, a FLAG tag, a Myc tag, or a 6His tag.

In another specific embodiment, the PD-L1 binding molecule is a monomer, dimer, or multimer.

In the third aspect, the present invention provides a humanized antibody, wherein the humanized antibody is derived from the nanobody of the first aspect.

In another preferred embodiment, the humanized antibody has the amino acid sequence shown in any one of SEQ ID NOs: 23-42.

In another preferred embodiment, the humanized antibody has the amino acid sequence shown in any one of SEQ ID NOs: 30, 33, 36, 40, and 41.

In another preferred embodiment, the humanized antibody has the amino acid sequence shown in SEQ ID NO: 30 or 40.

In the fourth aspect, the present invention provides a chimeric antigen receptor (CAR) comprising an extracellular domain that comprises an anti-PD-L1 nanobody of the first aspect, an anti-PD-L1 binding molecule of the second aspect, or a humanized antibody of the third aspect.

In another preferred embodiment, the extracellular domain further comprises a signal peptide.

In another preferred embodiment, the extracellular domain further comprises other exogenous proteins.

In another preferred embodiment, the CAR has a structure shown in Formula Ia:

L-Nb-H-TM-C-CD3ζ (Ia)

Wherein,
L is absent or a signal peptide sequence;
Nb is a specific binding domain;
H is absent or a hinge region;
TM is a transmembrane domain;
C is a co-stimulatory signaling domain;
CD3ζ is a cytoplasmic signaling sequence derived from CD3ζ (including the wild-type, or a mutant/modification thereof);
the "-" represents a linker peptide or a peptide bond.

In another preferred embodiment, L is a signal peptide respectively selected from following proteins: CD8, GM-CSF, CD4, CD28, CD137, or a mutant/modification thereof, or combinations thereof.

In another preferred embodiment, Nb targets PD-L1.

In another preferred embodiment, Nb is an anti-PD-L1 nanobody.

In another preferred embodiment, H is a hinge region selected from following proteins: CD8, CD28, CD137, IgG, or combinations thereof.

In another preferred embodiment, H is the hinge region of human IgG1 Fc.

In another preferred embodiment, TM is a transmembrane domain selected from following proteins: CD28, CD3 epsilon, CD45, CD4, CD5, CD8, CD9, CD16, CD22, CD33, CD37, CD64, CD80, CD86, CD134, CD137, CD154, CD278, CD152, CD279, CD233, or a mutant/modification thereof, or combinations thereof.

In another preferred embodiment, C is a co-stimulatory signaling domain selected from following proteins: OX40, CD2, CD7, CD27, CD28, CD30, CD40, CD70, CD134, 4-1BB (CD137), PD-1, Dap10, LIGHT, NKG2C, B7-H3, ICAM-1, LFA-1 (CD11a/CD18), ICOS (CD278), NKG2D, GITR, OX40L, 2B4, TLR, or a mutant/modification thereof, or combinations thereof.

In another preferred embodiment, the CAR comprises an intracellular domain of a cytokine.

In another preferred embodiment, the cytokine includes: interleukin (IL), interferon (IFN), tumor necrosis factor (TNF), colony-stimulating factor (CSF), growth factor, chemokine, or a combination thereof; and preferably, the cytokine is an interleukin.

In another preferred embodiment, the CAR comprises an intracellular domain of an interleukin.

In another preferred embodiment, the interleukin is selected from IL-12, IL-2, IL-15, IL-21, or a combination thereof; preferably, IL-12.

In the fifth aspect of the present invention, a fusion protein is provided, which comprises:
(Z1) a first protein, wherein the first protein includes: an anti-PD-L1 nanobody of the first aspect of the present invention, an anti-PD-L1 binding molecule of the second aspect of the present invention, or a humanized antibody or an active fragment thereof of the third aspect of the present invention;
(Z2) a second protein, wherein the second protein includes an Fc fragment or a cytokine; and
(Z3) an optional linker located between the first protein and the second protein.

In another preferred embodiment, the cytokine includes: interleukin (IL), interferon (IFN), tumor necrosis factor (TNF), colony-stimulating factor (CSF), growth factor, chemokine, or a combination thereof.

In another preferred embodiment, the second protein is an interleukin.

In another preferred embodiment, the interleukin is selected from IL-12, IL-2, IL-15, IL-21, or a combination thereof.

In another preferred embodiment, the interleukin is IL-12.

In another preferred embodiment, the Fc fragment is an Fc fragment of human IgG.

In the sixth aspect, a nucleic acid molecule is provided in the present invention, which comprises an encoding nucleic acid of the anti-PD-L1 nanobody or the heavy chain variable region VHH of the first aspect, the anti-PD-L1 binding molecule of the second aspect, the humanized antibody or an active fragment thereof of the third aspect, the chimeric antigen receptor of the fourth aspect, or the fusion protein of the fifth aspect.

In a specific embodiment, the nucleic acid molecule may encode the anti-PD-L1 nanobody, the heavy chain variable region VHH, the PD-L1 binding molecule, the humanized antibody, the chimeric antigen receptor, or the fusion protein, and may be an RNA, DNA, or cDNA.

In the seventh aspect, a recombinant expression vector is provided in the present invention, which comprises the nucleic acid molecule of the sixth aspect.

In a specific embodiment, the expression vector is selected from the group consisting of: DNA, RNA, viral vector, plasmid, transposon, any one of other gene transfer systems, or a combination of at least two thereof. Preferably, the expression vector comprises a viral vector, such as any one or a combination of at least two of lentiviruses, adenoviruses, AAV viruses, or retroviruses.

In another specific embodiment, the expression vector is selected from the group consisting of : a pTomo lentiviral vector, plenti, pLVTH, pLJM1, pHCMV, pLBS.CAG, pHR, pLV, pComb3XSS, or the like.

In another specific embodiment, the expression vector further comprises elements selected from promoters, transcriptional enhancer elements WPRE, long terminal repeat sequences LTR, or the like.

In the eighth aspect, a recombinant cell is provided in the present invention, which comprises the nucleic acid molecule of the sixth aspect or the recombinant expression vector of the seventh aspect.

Preferably, the nucleic acid molecule is integrated into the genome of the recombinant cell.

Preferably, the starting cell of the recombinant cell is a prokaryotic cell or a eukaryotic cell, further selected from the group consisting of Escherichia coli, a yeast cell, and a mammalian cell.

In the nineth aspect, a method for preparing the anti-PD-L1 nanobody of the first aspect is provided in the present invention, which comprises:
inserting a gene encoding the anti-PD-L1 nanobody of the first aspect into an expression vector to obtain a recombinant vector; introducing the recombinant vector into a host cell for culture; and isolating and obtaining the anti-PD-L1 nanobody from the culture.

In the tenth aspect, a use of the anti-PD-L1 nanobody of the first aspect, the anti-PD-L1 binding molecule of the second aspect, the humanized antibody or an active fragment thereof of the third aspect, the chimeric antigen receptor of the fourth aspect, or the fusion protein of the fifth aspect in targeted binding to PD-L1 is provided.

In the present invention, a broad application prospect is provided by screening and preparing anti-PD-L1 nanobodies with high specificity and affinity, such as *in vitro* immunological detection for purposes other than disease diagnosis and/or treatment, research on the fundamental behavior of PD-L1, and so on. Additionally, they can be used to prepare binding agents that bind to PD-L1, wherein the binding agents include: drugs for the prevention and/or treatment of PD-L1-overexpressing diseases; and/or reagents for detecting PD-L1-overexpressing diseases.

In the eleventh aspect, a use of the anti-PD-L1 nanobody of the first aspect, the anti-PD-L1 binding molecule of the second aspect, the humanized antibody or an active fragment thereof of the third aspect, the chimeric antigen receptor of the fourth aspect, or the fusion protein of the fifth aspect, the nucleic acid molecule of the sixth aspect, the recombinant vector of the seventh aspect, or the recombinant cell of the eighth aspect, in the preparation of a medicament for the targeted therapy of PD-L1-related diseases is provided in the present invention.

Preferably, the PD-L1-related disease is a PD-L1-overexpressing disease.

Preferably, the PD-L1-overexpressing disease is a PD-L1-expressing tumor.

Preferably, the PD-L1-expressing tumor includes but is not limited to: gastric cancer, lung cancer, liver cancer, osteosarcoma, breast cancer, pancreatic cancer, lymphoma, ovarian cancer, esophageal cancer, bladder (urothelial) cancer, melanoma, Merkel cell carcinoma, and the like.

In the 12^{th} aspect, an immunoconjugate is provided in the present invention, which comprises: the anti-PD-L1 nanobody of the first aspect; the anti-PD-L1 binding molecule of the second aspect; the humanized antibody or an active fragment thereof of the third aspect; or the fusion protein of the fifth aspect and a conjugation moiety, and the conjugation moiety is selected from any one or a combination of at least two of a protein, a small molecule compound, a fluorophore, a radioisotope, a contrast agent, or a fatty acid.

Preferably, the anti-PD-L1 nanobody is conjugated to the conjugation moiety via a chemical bond or a linker.

In a specific embodiment, the radioactive isotope comprises:
(i) a diagnostic isotope selected from any one or a combination of at least two of Tc-99m, Ga-68, F-18, I-123, I-125, I-131, In-111, Ga-67, Cu-64, Zr-89, C-11, Lu-177, or Re-188,; and/or
(ii) a therapeutic isotope selected from any one or a combination of at least two of Lu-177, Y-90, Ac-225, As-211, Bi-212, Bi-213, Cs-137, Cr-51, Co-60, Dy-165, Er-169, Fm-255, Au-198, Ho-166, I-125, 1-131, Ir-192, Fe-59, Pb-212, Mo-99, Pd-103, P-32, K-42, Re-186, Re-188, Sm-153, Ra-223, Ru-106, Na-24, Sr-89, Tb-149, Th-227, Xe-133, Yb-169, or Yb-177.

In the 13^{th} aspect, a pharmaceutical composition is provided in the present invention, comprising any one or a combination of at least two of the following: the anti-PD-L1 nanobody of the first aspect; the anti-PD-L1 binding molecule of the second aspect; the humanized antibody or an active fragment thereof of the third aspect; the chimeric antigen receptor of the fourth aspect; the fusion protein of the fifth aspect; the nucleic acid molecule of the sixth aspect; the recombinant vector of the seventh aspect; the recombinant cell of the eighth aspect; or the immunoconjugate of the 12^{th} aspect.

In the 14^{th} aspect, there is provided an engineered immune cell, which comprise the expression vector of the seventh aspect of the present invention, or has an exogenous nucleic acid molecule of the sixth aspect of the present invention integrated into the genome thereof, or expresses the chimeric antigen receptor of the fourth aspect of the present invention.

In another preferred embodiment, the engineered immune cell is selected from the group consisting of:
(i) a chimeric antigen receptor αβ T cell (CAR-T cell);
(ii) a chimeric antigen receptor γδ T cell (CAR-T cell);
(iii) a chimeric antigen receptor NKT cell (CAR-NKT cell);
(iv) a chimeric antigen receptor NK cell (CAR-NK cell).

In another preferred embodiment, the engineered immune cell includes an autologous or allogeneic αβ T cell, γδ T cell, NKT cell, NK cell, or a combination thereof.

In another preferred embodiment, the engineered immune cell is a CAR-T cell.

In the 15^{th} aspect, there is provided a kit, which comprises:
(1) a first container, wherein the first container contains: the anti-PD-L1 nanobody of the first aspect of the present invention; the anti-PD-L1 binding molecule of the second aspect; the humanized antibody or an active fragment thereof of the third aspect; the chimeric antigen receptor of the fourth aspect; the immunoconjugate of the thirteenth aspect of the present invention; the recombinant cell of the eighth aspect of the present invention; or a combination thereof; and/or
(2) a second container, wherein the second container contains a secondary antibody against the contents of the first container;
or
the kit comprises a detection plate, which comprises: a substrate (support plate) and a test strip, and the test strip comprises: the anti-PD-L1 nanobody of the first aspect of the present invention; the anti-PD-L1 binding molecule of the second aspect; the humanized antibody or an active fragment thereof of the third aspect; the chimeric antigen receptor of the fourth aspect; the immunoconjugate of the thirteenth aspect of the present invention, or a combination thereof.

In another preferred embodiment, the kit further comprises an instruction manual, which specifies that the kit is used for non-invasively detecting PD-L1 expression in a subject.

In another preferred embodiment, the kit is used for detecting a disease with high PD-L1 expression.

In another preferred embodiment, the disease with high PD-L1 expression includes but is not limited to: a solid tumor, hematological tumor, immune disease, or a combination thereof.

In another preferred embodiment, the disease with high PD-L1 expression includes but is not limited to: gastric cancer, lung cancer, liver cancer, osteosarcoma, breast cancer, pancreatic cancer, lymphoma, ovarian cancer, esophageal cancer, bladder (urothelial) cancer, melanoma, Merkel cell carcinoma, etc., or a combination thereof.

In the 16^{th} aspect of the present invention, there is provided a method for preventing and/or treating a PD-L1-related disease, the method comprising administering to a subject in need thereof an anti-PD-L1 nanobody of the first aspect of the present invention; an anti-PD-L1 binding molecule of the second aspect; a humanized antibody or an active fragment thereof of the third aspect; a chimeric antigen receptor of the fourth aspect; a fusion protein of the fifth aspect; an immunoconjugate of the thirteenth aspect; a pharmaceutical composition of the twelfth aspect of the present invention; or an engineered immune cell of the fourteenth aspect of the present invention, or a combination thereof.

In another preferred embodiment, the PD-L1-related disease is a disease with high PD-L1 expression.

In another preferred embodiment, the subject includes mammals, such as humans.

In another preferred embodiment, the disease with high PD-L1 expression includes but is not limited to: a solid tumor, hematological tumor, immune disease, or combinations thereof.

In another preferred embodiment, the disease with high PD-L1 expression includes but is not limited to: gastric cancer, lung cancer, liver cancer, osteosarcoma, breast cancer, pancreatic cancer, lymphoma, ovarian cancer, esophageal cancer, bladder (urothelial) cancer, melanoma, Merkel cell carcinoma, etc., or combinations thereof.

In another preferred embodiment, the method may be used in combination with other therapeutic methods.

In another preferred embodiment, the other therapeutic methods include chemotherapy, radiotherapy, targeted therapy, and the like.

In the 17^{th} aspect of the present invention, there is provided a diagnostic method for PD-L1-related diseases, comprising steps of:
(i) obtaining a sample from a subject to be diagnosed, and contacting the sample with an anti-PD-L1 nanobody of the first aspect of the present invention, an anti-PD-L1 binding molecule of the second aspect, a humanized antibody or an active fragment thereof of the third aspect, an immunoconjugate of the tenth aspect of the present invention, or a combination thereof; and
(ii) detecting whether an antigen-antibody complex is formed, wherein the formation of the complex indicates that the subject is a confirmed patient with a disease with high PD-L1 expression.

In another preferred embodiment, the PD-L1-related disease is a disease with high PD-L1 expression.

In another preferred embodiment, the sample is a blood sample, a pharyngeal swab sample, or a sample from other tissues or organs.

In another preferred embodiment, the disease with high PD-L1 expression includes but is not limited to: a solid tumor, hematological tumor, immune disease, or a combination thereof.

In another preferred embodiment, the disease with high PD-L1 expression includes but is not limited to: gastric cancer, lung cancer, liver cancer, osteosarcoma, breast cancer, pancreatic cancer, lymphoma, ovarian cancer, esophageal cancer, bladder (urothelial) cancer, melanoma, Merkel cell carcinoma, etc., or combinations thereof.

In the present invention, a PD-L1 heavy chain single-domain antibody (nanobody) with high affinity and specificity is screened and prepared. Based on this, it can be further conjugated with a chemical label, biological label, small molecule compound, protein, and the like to prepare an immunological conjugate for use in the fields of immunoassays and therapeutics. For example, the chemical label may include an isotope, immunotoxin, and/or chemotherapeutic drug; the biological label may include biotin, avidin, or enzyme label; the small molecule compound may include, but not limited to, a drug or toxin with known or potential therapeutic or adjuvant therapeutic effects for tumors or autoimmune diseases; the imaging agent may include gold nanoparticles/nanorods or magnetic nanoparticles; and the protein may include, but not limited to, an antibody Fc, HRP, antibodies, enzymes, cytokines, viral coat proteins, VLPs, and other bioactive proteins or peptides.

Preferably, the protein includes IL-12 protein.

### Description of Drawings

Figure 1 shows the SDS-PAGE validation for the purity of purified PD-L1;
Figure 2 shows the SDS-PAGE identification for the purified nanobody;
Figure 3 shows the identification for the antigen recombinant protein;
Figure 4 shows the ELISA detection for the binding of candidate PD-L1 nanobody to human PD-L1 protein (Part 1/2);
Figure 5 shows the ELISA detection for the binding of candidate PD-L1 nanobody to human PD-L1 protein (Part 2/2);
Figure 6 shows the SPR analysis for determining the affinity of nanobody binding to PD-L1 at a single concentration;
Figure 7 shows the FACS detection for the binding activity of PD-L1 nanobody with PD-L1 expressed on Raji cells;
Figure 8 shows the ELISA detection of PD-L1 nanobody blockade of PD-1/PD-L1 interaction;
Figure 9 shows the characterization for the blocking activity of the nanobody on the surface of Raji cells;
Figure 10 shows the SDS-PAGE identification for the protein purity of anti-PD-L1-Fc nanobody;
Figure 11 shows the binding of the NbsPD-L1-Fc fusion protein to human PD-L1 protein;
Figure 12 shows the ELISA detection for the binding capacity of the Nbs-PD-L1-Fc fusion protein to PD-L1 protein;
Figure 13 shows the SPR analysis of the NbsPD-L1-Fc fusion protein affinity ;
Figure 14 shows the FACS detection for the binding activity of NbsPDL1-1-Fc to Raji-PDL1/Raji cells;
Figure 15 shows the binding specificity of the PD-L1 nanobody to the PDL1 protein;
Figure 16A shows the identification of the PDL1-1 humanized single-domain antibodies (PDL1-1v4, PDL1-1v7, PDL1-1v9, PDL1-1v1, and PDL1-1v2);
Figure 16B shows the identification of the PDL1-1 humanized single-domain antibodies (PDL1-1v3, PDL1-1v10, PDL1-1v8, PDL1-1v5, PDL1-1v6, PDL1-1v10, PDL1-1v13, PDL1-1v19, and PDL1-1v17);
Figure 16C shows the identification of the PDL1-1 humanized single-domain antibodies (PDL1-lvl1, PDL1-1v14, PDL1-1v16, PDL1-1v15, PDL1-1v12, and PDL1-1v18);
Figure 17 shows the detection for the binding affinity of the PDL1-1 humanized single-domain antibodies;
Figure 18 shows the detection for the binding affinity of the PDL1-1 humanized single-domain antibodies;
Figure 19 shows the detection for the binding affinity of the PDL1-1 humanized single-domain antibodies;
Figure 20 shows the detection for the binding affinity of the PDL1-1 humanized single-domain antibodies;
Figure 21 shows the detection for the blocking activity of the PDL1-1 humanized single-domain antibodies;
Figure 22 shows the detection for the blocking activity of the PDL1-1 humanized single-domain antibodies at the cellular level;
Figure 23 shows the detection for the activation of PBMC by the PDL1 single-domain antibodies and its humanized antibodies;
Figure 24 shows the detection for the activation of PBMC by the PDL1 single-domain antibodies and its humanized antibodies;
Figure 25 shows the detection for the cytotoxicity of the PDL1-1 humanized heavy-chain single-domain antibodies against target cells;
Figure 26 shows the schematic of the fusion protein expression constructs;
Figure 27 shows the gel electrophoresis analysis of fusion proteins;
Figure 28 shows the PD-L1 binding activity of Fusion Proteins 1-4;
Figure 29 shows the PD-L1 binding activity of Fusion Proteins 5-9;
Figure 30 shows the sensorgram and binding curves of Fusion Proteins 1-4;
Figure 31 shows the sensorgram and binding curves of Fusion Proteins 5-9;
Figure 32 shows the enhanced killing effect of effector cells on target cells mediated by Fusion Proteins 4, 7, and 8.

### Modes for carrying out the invention

The present inventors conducted extensive and in-depth research and a large number of screenings, and unexpectedly obtained anti-PD-L1 nanobodies with high affinity and high specificity for the first time. Fusion proteins prepared by using the anti-PD-L1 nanobodies and IL-12 protein are capable of both targeting tumor lesions and releasing the IL-12 cytokine, thereby stimulating immune cells to exert their immune-killing functions. The present invention provides better diagnostic approaches for treating PD-L1-related diseases, such as tumors. The present invention is completed based on these findings.

### Terms

For clarity, certain terms are defined herein. As used in this application, unless explicitly specified otherwise, the following terms shall have the meanings indicated below.

The term "about" may refer to a value or composition within an acceptable margin of error for a specific value or composition as determined by a skilled person in the art, which may depend in part on how the value or composition is measured or determined.

The term "administration" refers to physically introducing the product of the present invention into a subject using any of various methods and delivery systems known known to those skilled in the art, including, but not limited to, intravenous, intra-tumoral, intramuscular, subcutaneous, intraperitoneal, intraspinal, or other parenteral administration routes, such as via injection or infusion.

In a specific embodiment, the amino acid sequences of CDR1, CDR2, and CDR3 of the nanobody of the present invention, as well as the amino acid sequence of the VHH chain, are respectively shown in the following table:

| CDR1 Number of amino acid sequence | CDR2 Number of amino acid sequence | CDR3 Number of amino acid sequence | VHH chain Number of amino acid sequence |
|---|---|---|---|
| 1 | 2 | 3 | 13 |
| 4 | 5 | 6 | 14 |
| 7 | 8 | 9 | 15 |
| 10 | 11 | 12 | 16 |

As used herein, the term "antibody" or "immunoglobulin" refers to a heterotetrameric glycoprotein with approximately 150,000 Daltons and identical structural features, which consists of two identical light chains (L) and two identical heavy chains (H). Each light chain is linked to a heavy chain via a covalent disulfide bond, whereas the number and position of disulfide bonds between heavy chains differs among various immunoglobulin isotypes. Both heavy and light chains also contain regularly spaced intrachain disulfide bonds. There is a variable region (VH) at one end of each heavy chain, followed by multiple constant regions. There is a variable region (VL) at one end of each light chain, while there is a constant region at the other end; the constant region of the light chain corresponds to the first constant region of the heavy chain, and the variable region of the light chain corresponds to the variable region of the heavy chain. Specific amino acid residues form an interface between the variable regions of the light and heavy chains.

As used herein, the terms "single-domain antibody", "VHH", "Nanobody" and "single-domain antibody (single domain antibody, sdAb; or nanobody)" have the same meaning and are interchangeable. They refer to a single-domain antibody (VHH) constructed by cloning the variable region of an antibody heavy chain, which consists of only one heavy chain variable region and represents the smallest fully functional antigen-binding fragment. Generally, an antibody naturally lacking a light chain and heavy chain constant region 1 (CH1) is first obtained, followed by cloning of the variable region of the antibody heavy chain to construct a single-domain antibody (VHH) composed of only one heavy chain variable region.

In the present invention, the terms "the nanobody of the present invention," "the antibody of the present invention," "the protein of the present invention," and "the polypeptide of the present invention" are interchangeable and all refer to polypeptides that specifically bind to PD-L1, such as proteins or polypeptides comprising a heavy chain variable region. They may or may not contain an initiating methionine.

As used herein, the term "variable" refers to portions of the variable regions of antibodies that differ in sequence, which enable various specific antibodies to bind to and exhibit specificity for their particular antigens. However, variability is not uniformly distributed across the entire antibody variable region. It is concentrated in three segments known as complementary determining regions (CDRs) or hypervariable regions within the light and heavy chain variable regions. The relatively conserved portions of the variable regions are referred to as framework regions (FRs). The variable regions of natural heavy and light chains each contain four FR regions, which generally adopt a β-sheet conformation. These FR regions are linked by three CDRs that form connecting loops; and in some cases, partial β-sheet structures may also form. The CDRs in each chain are closely positioned via the FR regions and, together with the CDRs of the other chain, form the antigen-binding site of an antibody (see Kabat et al., NIH Publ. No. 91-3242, Vol. I, pp. 647-669 (1991)). The constant regions do not directly participate in antibody-antigen binding, but they exhibit distinct effector functions, such as involvement in antibody-dependent cellular cytotoxicity.

As used herein, the terms "hypervariable region", "hyper-variable region", "complementarity-determining region" and "complementarity determining region (CDR)" may be used interchangeably. In a preferred embodiment of the present invention, the heavy chain variable region of the nanobody or antibody comprises three complementarity determining regions: CDR1, CDR2, and CDR3. In another preferred embodiment of the present invention, the heavy chain of the nanobody or antibody comprises a heavy chain variable region and a heavy chain constant region.

The antigen-binding properties of antibodies are generally described by three specific regions located in the variable region of the heavy chain, known as complementarity determining regions (CDRs). These CDRs divide the segment into four framework regions (FRs). The amino acid sequences of the four FRs are relatively conserved and do not directly participate in binding reactions. These CDRs form loop structures, and come spatially close to each other through the β-sheets formed by the intervening FRs. The CDRs on the heavy chain and the corresponding CDRs on the light chain together constitute the antigen-binding site of the antibody. By comparing the amino acid sequences of antibodies of the same type, it is possible to determine which amino acids form the FR or CDR regions.

The heavy chain variable regions of the nanobodies or antibodies of the present invention are critical, since at least a portion of them is involved in antigen binding. Therefore, the present invention encompasses molecules having heavy chain variable regions of antibodies with CDRs, provided that their CDRs exhibit homology of greater than 90% (preferably greater than 95%, and most preferably greater than 98%) to the CDRs identified herein.

In a specific embodiment, the amino acid sequences of CDR1, CDR2, and CDR3 of the nanobodies of the present invention, as well as the amino acid sequence of the VHH chain, are respectively shown in the following table:

| CDR1 Number of amino acid sequence | CDR2 Number of amino acid sequence | CDR3 Number of amino acid sequence | VHH chain Number of amino acid sequence |
|---|---|---|---|
| 1 | 2 | 3 | 13 |
| 4 | 5 | 6 | 14 |
| 7 | 8 | 9 | 15 |
| 10 | 11 | 12 | 16 |

As is known to a skilled person, immunoconjugates and fusion expression products include: conjugates formed by coupling drugs, toxins, cytokines (Cytokine), radionuclides, enzymes, and other diagnostic or therapeutic molecules with the antibody or a fragment thereof of the present invention. The present invention further includes cell surface markers or antigens conjugated to the nanobody targeting CD38 or a fragment thereof.

In the present invention, other proteins or fusion expression products comprising the antibody of the present invention are also provided. Specifically, the present invention includes any protein or protein conjugate as well as fusion expression product (i.e., immunoconjugates and fusion expression products) comprising a heavy chain with a variable region, provided that such a variable region is identical to or at least 90% homologous, preferably at least 95% homologous to the heavy chain variable region of the antibody of the present invention.

The term "humanized antibody" as used herein has the meaning commonly understood by a skilled person, which refers to an antibody obtained by humanizing an alpaca-derived antibody, thereby reducing the immunogenicity of the alpaca-derived antibody. A skilled person will know how to perform humanization to obtain a humanized antibody based on the anti-PD-L1 nanobody of the present invention. In a specific embodiment, the humanized antibody of the present invention has the amino acid sequence shown in any one of SEQ ID NOs: 23-42; preferably any one of SEQ ID NOs: 30, 33, 36, 40, 41; and more preferably the amino acid sequence shown in SEQ ID NO: 30 or 40.

To enhance the affinity of the humanized antibody, a skilled person may perform back mutations on a humanized antibody to obtain a humanized antibody with improved affinity.

Based on the teachings of the present invention, a skilled person will also know how to obtain active fragments of the antibodies of the present invention, i.e., antibody fragments having the same or similar immunological activities as the antibodies of the present invention. Therefore, the present invention includes not only intact antibodies but also fragments of an antibody having immunological activity or fusion proteins formed from an antibody with other sequences. For example, the present invention further includes fragments, derivatives, and analogs of the antibodies.

As used herein, the terms "fragment", "derivative" and "analog" refer to polypeptides that substantially retain the same biological functions or activities as the antibody of the present invention. The polypeptide fragments, derivatives, or analogs of the present invention include, but are not limited to: (i) polypeptides having one or more conservative or non-conservative amino acid residues (preferably conservative amino acid residues) substituted, wherein such substituted amino acid residues may or may not be encoded by genetic codes; or (ii) polypeptides having substituent groups at one or more amino acid residues; or (iii) polypeptides formed by fusion of a mature polypeptide with another compound (e.g., a compound that extends the half-life of the polypeptide, such as polyethylene glycol); or (iv) polypeptides formed by fusion of additional amino acid sequences to this polypeptide sequence (e.g., leader sequences, secretory sequences, sequences used for purifying the polypeptide, prosequences, or fusion proteins with a 6His tag). Under the teachings herein, these fragments, derivatives, and analogs fall within the purview of a skilled person.

The term "antibodies of the present invention" includes not only polypeptides comprising the aforementioned CDR regions and having PD-L1 protein binding activities, but also variants of such polypeptides that possess the same function as the antibodies of the present invention and comprise the aforementioned CDR regions. These variants include (but are not limited to): deletions, insertions, and/or substitutions of one or more (typically 1-50, preferably 1-30, more preferably 1-20, and most preferably 1-10) amino acids, as well as the addition of one or several (typically no more than 20, preferably no more than 10, and more preferably no more than 5) amino acids at the C-terminus and/or N-terminus. For example, in the art, substitutions with amino acids having similar or comparable properties generally do not alter the function of a protein. For another example, the addition of one or several amino acids at the C-terminus and/or N-terminus typically does not affect a protein's function. This term also encompasses active fragments and active derivatives of the antibodies of the present invention.

Variants of the polypeptide include: homologous sequences, conservative variants, allelic variants, naturally occurring mutants, induced mutants, proteins encoded by a DNA that can hybridize with the DNA encoding the antibody of the present invention under high or low stringency conditions, as well as polypeptides or proteins obtained using antisera against the antibody of the present invention.

The present invention also provides other polypeptides, such as fusion proteins comprising the antibody or a fragment thereof. In addition to a nearly full-length polypeptide, the present invention also includes fragments of the antibody of the present invention. Generally, such fragments comprise at least about 50 contiguous amino acids of the antibody of the present invention, preferably at least about 50 contiguous amino acids, more preferably at least about 80 contiguous amino acids, and most preferably at least about 100 contiguous amino acids.

In the present invention, the "conservative variants of the antibody of the present invention" refer to polypeptides formed by replacing up to 10, preferably up to 8, more preferably up to 5, and most preferably up to 3 amino acids in the amino acid sequence of the antibody of the present invention with amino acids of similar or analogous properties. These conservative variant polypeptides are preferably generated by amino acid substitutions according to the table below.

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides a polynucleotide molecule encoding the aforementioned antibody or a fragment or fusion protein thereof. The polynucleotides of the present invention may be in the form of DNA or RNA. The DNA form includes cDNA, genomic DNA, or artificially synthesized DNA. The DNA may be single-stranded or double-stranded. The DNA may be the coding strand or the non-coding strand.

The polynucleotides encoding the mature polypeptide of the present invention include: a coding sequence that encodes only the mature polypeptide; a coding sequence of the mature polypeptide and various additional coding sequences; a coding sequence of the mature polypeptide (and optionally additional coding sequences) as well as non-coding sequences.

The term "polynucleotide encoding a polypeptide" may refer to a polynucleotide that includes the coding sequence for the polypeptide, or may also include additional coding and/or non-coding sequences.

The present invention also relates to polynucleotides that hybridize with the aforementioned sequences and have at least 50%, preferably at least 70%, more preferably at least 80% sequence identity between the two sequences. The invention particularly relates to polynucleotides that are capable of hybridizing with the polynucleotides described herein under a stringent condition. In the present invention, "a stringent condition" refers to: (1) hybridization and washing under a low ionic strength and high temperature, such as 0.2×SSC, 0.1% SDS, 60°C; or (2) hybridization in the presence of a denaturing agent, such as 50% (v/v) formamide, 0.1% calf serum / 0.1% Ficoll, 42°C, etc.; or (3) hybridization occurs only when the sequence identity between the two sequences is at least 90%, more preferably at least 95%. Furthermore, the polypeptides encoded by the hybridizable polynucleotides possess the same biological function and activity as the mature polypeptide.

The full-length sequence or fragments thereof of the nucleotide of the antibody according to the present invention can generally be obtained by PCR amplification, recombinant methods, or artificial synthesis. One feasible approach is to synthesize the relevant sequences using artificial synthesis, especially when the fragment is in relatively short length. Typically, long sequences can be obtained by first synthesizing multiple small fragments and then ligating them together. In addition, the coding sequence of the heavy chain can be fused with an expression tag (such as 6His) to form a fusion protein.

Once the relevant sequences are obtained, they can be produced in large quantities using recombinant methods. This usually involves cloning them into a vector, transferring the vector into cells, and then isolating the relevant sequences from the proliferated host cells using conventional methods. The biological molecules (nucleic acids, proteins, etc.) involved in the present invention include those in isolated forms.

Currently, the DNA sequence encoding the protein of the present invention (or a fragment thereof, or a derivative thereof) can be entirely obtained through chemical synthesis. This DNA sequence can then be introduced into various existing DNA molecules (or vectors, for example) and cells known in the art. In addition, mutations can also be introduced into the protein sequence of the present invention via chemical synthesis.

The present invention also relates to vectors comprising the aforementioned appropriate DNA sequence as well as an appropriate promoter or control sequence. These vectors can be used to transform suitable host cells so that they are capable of expressing the protein.

The host cells may be prokaryotic cells, such as bacterial cells; or lower eukaryotic cells, such as yeast cells; or higher eukaryotic cells, such as mammalian cells. Representative examples include: bacterial cells such as *Escherichia coli* and *Streptomyces species,* or *Salmonella typhimurium*; fungal cells such as yeast; insect cells such as Drosophila S2 or Sf9; animal cells such as CHO, COS7, and 293 cells, among others.

The transformation of host cells with recombinant DNA can be performed using conventional techniques well known to a skilled person. When the host is a prokaryote, such as *Escherichia coli,* competent cells capable of taking up DNA can be harvested after the exponential growth phase and treated by the CaCl₂ method, the steps of which are well-known in the art. Another method is to use MgCl₂. If desired, the transformation can also be carried out by electroporation. When the host is a eukaryote, DNA transfection methods, such as calcium phosphate co-precipitation, conventional mechanical methods (e.g., microinjection, electroporation, liposome-mediated packaging), and others may be employed.

The obtained transformants can be cultured using conventional methods to express the polypeptides encoded by the genes of the present invention. Depending on the used host cell, the culture medium may be selected from various conventional media. Culturing is carried out under conditions suitable for the growth of the host cells. Once the host cells have reached an appropriate cell density, the selected promoter is induced by an appropriate method (e.g., temperature shift or chemical induction), and the cells are cultured for another period of time.

The recombinant polypeptides in the above methods can be expressed intracellularly, on the cell membrane, or secreted extracellularly. If needed, the recombinant proteins can be isolated and purified using various separation methods based on their physical, chemical, and other properties. These methods are well known to a skilled person. Examples of such methods include, but are not limited to: conventional renaturation treatment, treatment with protein precipitation agents (such as salting-out methods), centrifugation, osmotic lysis, ultrafiltration, ultracentrifugation, molecular sieve chromatography (gel filtration), adsorption chromatography, ion exchange chromatography, high-performance liquid chromatography (HPLC), and other various liquid chromatography techniques, as well as combinations of these methods.

The nanobodies, antibodies, or humanized antibodies according to the present invention may be used alone or conjugated or coupled with detectable markers (for diagnostic purposes), therapeutic agents, PK (protein kinase) modifying moieties, or any combination of the above.

Detectable markers for diagnostic purposes include, but are not limited to: fluorescent or luminescent markers, radioactive markers, MRI (Magnetic Resonance Imaging) or CT (Computed Tomography) contrast agents, or enzymes capable of generating detectable products.

Therapeutic agents that may be conjugated or coupled with the antibodies of the present invention include, but are not limited to: 1. Radionuclides; 2. Biological toxins; 3. Cytokines, such as IL-2, etc.; 4. Gold nanoparticles/nanorods; 5. Virus particles; 6. Liposomes; 7. Nanomagnetic particles; 8. Prodrug-activating enzymes (e.g., DT-diaphorase (DTD) or biphenyl hydrolase-like protein (BPHL)), etc.

### PD-L1-related diseases

Based on the teachings of the present invention, a skilled person will understand that the nanobodies, active fragments or derivatives thereof of the present invention can be used for treating PD-L1-related diseases.

The PD-L1-related diseases refer to diseases with high PD-L1 expression. In a specific embodiment, the diseases with high PD-L1 expression are tumors expressing PD-L1; including but not limited to: gastric cancer, lung cancer, liver cancer, osteosarcoma, breast cancer, pancreatic cancer, lymphoma, ovarian cancer, esophageal cancer, bladder (urothelial) cancer, melanoma, Merkel cell carcinoma, and the like.

### Kit

The present invention also provides a kit comprising the anti-PD-L1 nanobody or antibody or humanized antibody (or a fragment thereof) or detection plate of the present invention. In a preferred embodiment, the kit further comprises a container, an instruction manual, a buffer, and the like.

The present invention also provides a detection kit for detecting the level of PD-L1 protein, which includes the antibody of the present invention that recognizes the PD-L1 protein, a lysis medium for dissolving the sample, as well as general reagents and buffers required for detection, such as various buffers, detection labels, detection substrates, and the like. The detection kit may be an *in vitro* diagnostic device.

### Pharmaceutical composition

Based on the antibody of the present invention, the present invention further provides a pharmaceutical composition comprising, as an active ingredient, the anti-PD-L1 nanobody, a PD-L1 binding molecule, a humanized antibody, a chimeric antigen receptor, a fusion protein, a recombinant protein, a host cell, an engineered immune cell, an immunoconjugate of the present invention, or a combination thereof. The pharmaceutical composition of the present invention may further comprise an optional pharmaceutically acceptable carrier.

Typically, these substances can be formulated in a non-toxic, inert, and pharmaceutically acceptable aqueous carrier medium, with the pH generally being around 5-8, preferably around 6-8, although the pH may vary depending on the nature of the substance being formulated and the condition to be treated. The prepared pharmaceutical composition can be administered via conventional routes, including (but not limited to): intraperitoneal, intravenous, or topical administration.

The pharmaceutical composition of the present invention contains a safe and effective amount (e.g., 0.001-99 wt%, preferably 0.01-90 wt%, more preferably 0.1-80 wt%) of the aforementioned antibody (or conjugate) of the invention, as well as a pharmaceutically acceptable carrier or excipient. Such carriers include (but are not limited to): saline, buffer solutions, glucose, water, glycerol, ethanol, and combinations thereof. The pharmaceutical formulation should match the method of administration. The pharmaceutical composition of the invention can be prepared in the form of an injectable, for example, by conventional methods using physiological saline or an aqueous solution containing glucose and other excipients. Pharmaceutical compositions, such as injections or solutions, are preferably prepared under sterile conditions. The dosage of the active ingredient is a therapeutically effective amount, for example, about 10 micrograms per kilogram of body weight to about 50 milligrams per kilogram of body weight per day. In addition, the polypeptides of the invention may also be used in combination with other therapeutic agents.

When using the pharmaceutical composition, a safe and effective amount of the immunoconjugate is administered to a mammal. Typically, such safe and effective amount is at least about 10 micrograms per kilogram of body weight, and in most cases does not exceed about 50 milligrams per kilogram of body weight. Preferably, the dosage is in a range of about 10 micrograms per kilogram of body weight to about 10 milligrams per kilogram of body weight. Of course, the specific dosage should also take into account factors, such as the route of administration and the patient's health condition, all of which are within the skill of a trained medical practitioner.

### Uses

As described above, the nanobodies of the present invention, active fragments or derivatives thereof, possess broad biological and clinical application value, applications of which relate to multiple fields, including the diagnosis and treatment of diseases related to the PD-L1 protein, basic medical research, and biological research. A preferred application is for the clinical diagnosis, prevention, and treatment targeting the PD-L1 protein.

The present invention also provides a method for stimulating an immune response mediated by T cells that targets a population of tumor cells or tissues in a mammal, comprising the step of administering the CAR-T cells of the present invention to the mammal.

In one embodiment, the present invention includes a type of cell therapy, wherein autologous T cells (or allogeneic donor cells) are isolated from a patient, activated, and genetically engineered to produce CAR-T cells, which are then infused back into the same patient. This method significantly reduces the likelihood of graft-versus-host disease, as the antigens are recognized by the T cells in an MHC-unrestricted manner. Furthermore, a single CAR-T can be used to treat all cancers expressing the target antigen. Unlike antibody therapies, CAR-T cells are capable of being replicated *in vivo*, leading to long-term persistence that can result in sustained tumor control.

In one embodiment, the CAR-T cells of the present invention can undergo stable *in vivo* expansion and persist for a period ranging from several months to several years. Additionally, the CAR-mediated immune response may be part of an adoptive immunotherapy procedure, wherein the CAR-T cells can induce a specific immune response against tumor cells that highly express the antigen recognized by the antigen-binding domain of the CAR. For example, the CAR-T cells of the present invention elicit a specific immune response against tumor cells with high PD-L1 expression. In a specific embodiment, the tumors with high PD-L1 expression include, but are not limited to: gastric cancer, lung cancer, liver cancer, osteosarcoma, breast cancer, pancreatic cancer, lymphoma, ovarian cancer, esophageal cancer, bladder (urothelial) cancer, melanoma, Merkel cell carcinoma, and the like.

Typically, the cells activated and expanded as described herein can be used for the treatment and prevention of diseases such as tumors. Accordingly, the present invention provides a method for treating a cancer, comprising administering to a subject in need thereof a therapeutically effective amount of the CAR-T cells of the present invention.

The CAR-T cells of the invention may be administered alone or as a pharmaceutical composition in combination with diluents and/or other components such as IL-2, IL-17, or other cytokines or cell populations. Briefly, the pharmaceutical composition of the invention may comprise the target cell population as described herein, in combination with one or more pharmaceutically or physiologically acceptable carriers, diluents, or excipients.

The pharmaceutical composition of the invention may be administered in a manner suitable for the disease to be treated (or prevented). The dosage and frequency of administration will be determined by factors, such as the patient's condition, the type and severity of the patient's disease, or may be determined through clinical trials.

When referring to an "immunologically effective amount", "anti-tumor effective amount", "tumor-inhibiting effective amount" or "therapeutic amount", the precise dosage of the composition of the present invention to be administered can be determined by a physician, taking into account the patient's (subject's) age, weight, tumor size, extent of infection or metastasis, and individual variations in condition. The pharmaceutical composition comprising the T cells described herein may be administered at a dosage of 10⁴ to 10⁹ cells per kilogram of body weight, preferably at a dosage of 10⁵ to 10⁷ cells per kilogram of body weight (including all integer values within this range). The T cell composition may also be administered multiple times at these dosages. The cells may be administered using infusion techniques well known in immunotherapy (see, for example, Rosenberg et al., New Engl. J. of Med. 319:1676, 1988). The optimal dosage and treatment regimen for a specific patient can be readily determined by monitoring the patient's disease indicators and adjusting the treatment accordingly by a skilled person in the medical field.

The composition can be administered in any convenient manner, including by spraying, injection, swallowing, infusion, implantation, or transplantation. The compositions described herein may be administered to a patient subcutaneously, intradermally, intratumorally, intranodally, intrathecally, intramuscularly, via intravenous injection, or intraperitoneally. In one embodiment, the T-cell composition of the present invention is administered to a patient by intradermal or subcutaneous injection. In another embodiment, the T-cell composition of the present invention is preferably administered by intravenous injection. The T-cell composition may be directly injected into a tumor, lymph node, or site of infection.

In certain embodiments of the present invention, the activated and expanded cells obtained using the methods described herein or other methods known in the art for expanding T cells to therapeutic levels are administered to a patient in combination with (e.g., before, simultaneously with, or after) any number of related therapeutic modalities, which include, but are not limited to, treatment with agents such as antiviral therapy, cidofovir, and interleukin-2; cytarabine (also known as ARA-C); or natalizumab therapy for MS patients, efalizumab therapy for psoriasis patients, or other treatments for PML patients. In further embodiments, the T cells of the invention may be used in combination with chemotherapy, radiation, immunosuppressive agents, such as cyclosporine, azathioprine, methotrexate, mycophenolate mofetil, and FK506, antibodies, or other immunotherapeutic agents. In yet further embodiments, the cell compositions of the invention are administered to a patient in combination with (e.g., before, simultaneously with, or after) bone marrow transplantation, chemotherapeutic agents, such as fludarabine, external beam radiation therapy (XRT), or cyclophosphamide. For example, in one embodiment, a subject may undergo a standard treatment with high-dosage chemotherapy followed by peripheral blood stem cell transplantation. In some embodiments, after transplantation, the subject receives an infusion of the expanded immune cells of the invention. In an additional embodiment, the expanded cells are administered either before or after surgery.

The aforementioned dosage for the treatment administered to patients will vary depending on the specific nature of the condition being treated and the recipient of the therapy. The dosage proportion for human administration can be implemented in accordance with practices accepted in the field. Typically, between 1×10⁵ and 1×10¹⁰ of the modified T cells of the present invention may be administered to a patient per treatment or per course of treatment, for example, by intravenous infusion.

### Major advantages of the present invention:

1. The anti-PD-L1 nanobody of the present invention exhibits high affinity and high specificity, enabling it to efficiently target PD-L1;
2. The anti-PD-L1 nanobody of the present invention is of a simple structure and readily to be prepared, thereby demonstrating broad application prospects in fields, such as immunoassays and tumor therapy.

To further elaborate the technical means adopted by the present invention and the produced effects, the following provides a more detailed description of the invention in conjunction with embodiments and accompanying drawings. It is to be understood that the specific embodiments described herein are intended only to explain the invention and do not limit the scope of the invention.

For techniques or conditions not specified in the embodiments, those commonly described in the literature of the field or according to product instructions are followed. Reagents or instruments for which the manufacturers are not specified are all conventional products that can be purchased through regular channels.

In the specific embodiments of the present invention, the definitions of abbreviations and key terms are as follows.

| abbreviation | Full English designation | Full Chinese designation |
|---|---|---|
| AHC | anti-human IgG (Fc) capture antibody | Anti-human Fc capture antibody |
| CDR | complementary determining region | Complementarity-determining region (CDR) |
| EC₅₀ | concentration for 50% of maximal effect | Half-maximal effective concentration |
| FBS | fetal bovine serum | Fetal bovine serum |
| Fc | fragment of crystalization | Crystallizable Fragment (Fc region of an antibody) |
| FITC | fluorescein isothiocyanate | Fluorescein isothiocyanate |
| HRP | horseradish peroxidase | Horseradish peroxidase |
| HSA | human serum albumin | Human serum albumin |
| IC₅₀ | half maximal inhibitory concentration | Half-maximal inhibitory concentration |
| IFN | interferon | Interferon |
| IgG | immunoglobulin G | Immunoglobulin G |
| kD | kilodalton | Kilodalton |
| mAb | monoclonal antibody | Monoclonal antibody |
| Nb | Nanobody | Nanobody |
| MFI | median fluorescent intensity | Median fluorescence intensity |
| NK | nature killer | Natural killer |
| nM | nanomole | Nanomolar |
| OD | optical density | Optical density |
| PBS | phosphate-buffered saline | Phosphate-buffered saline |
| PCR | polymerase chain reaction | Polymerase chain reaction |
| PEG | polyethylene glycol | Polyethylene glycol |
| SDS | sodium dodecyl sulface | Sodium dodecyl sulfate |
| SPR | surface plasmon resonance | Surface plasmon resonance |
| TMB | 3,3',5,5'-tetramethylbenzidine | 3,3',5,5'-Tetramethylbenzidine |

### Example 1

In this example, heavy-chain single-domain antibodies targeting PD-L1 were screened.

### 1. Preparation of Immunogenic Antigen

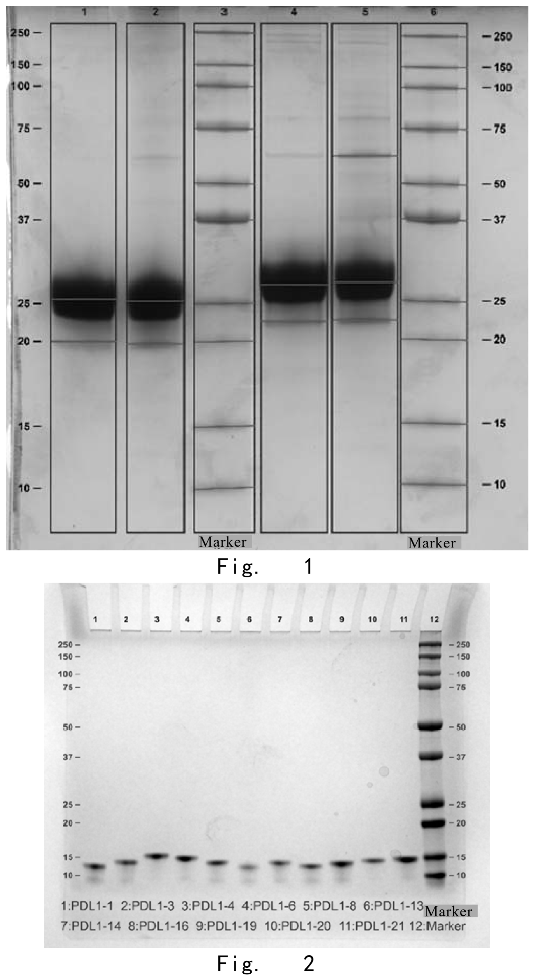

The extracellular domain (1-238) of the antigen PD-L1 was synthesized via gene synthesis, with a 6× his tag added to the C-terminus. It was then subcloned into the eukaryotic expression vector pcDNA3.1 to construct an expression vector for the recombinant protein PD-L1-His, the amino acid sequence of which is shown in SEQ ID NO. 17. The constructed PD-L1-His plasmid was extracted, sequenced, and transfected into FreeStyle^{™} 293-F cells. The cells were cultured in a shaking flask at 37°C, 120 rpm, and 5% CO₂ for 3.5 days. The supernatant was collected by centrifugation, filtered through a 0.45 µm membrane, and purified by affinity chromatography using HisTrap^{™} FF. The sample was eluted with 500 mM imidazole, and buffer exchange and concentration were performed using an ultrafiltration concentrator. Protein purity was assessed by SDS-PAGE, with the results shown in Figure 1. The electrophoretic position of the antigen protein matched expectations, with clear bands and high purity. No fragmentation or degradation bands were observed for the PD-L1 protein.

Lanes 1 and 2 showed the non-reduced form of the PD-L1 protein, lanes 4 and 5 showed the reduced form of the PD-L1 protein, and lanes 3 and 6 were protein molecular weight markers. The protein concentration was quantified using a spectrophotometer at 2 mg/mL.

SEQ ID NO.17:
MRIFAVFIFMTYWHLLNAFTVTVPKDLYVVEYGSNMTIECKFPVEKQLDLAALIVY
WEMEDKNIIQFVHGEEDLKVQHSSYRQRARLLKDQLSLGNAALQITDVKLQDAGVYRCM
ISYGGADYKRITVKVNAPYNKINQRILVVDPVTSEHELTCQAEGYPKAEVIWTSSDHQVLS
GKTTTTNSKREEKLFNVTSTLRINTTTNEIFYCTFRRLDPEENHTAELVIPELPLAHPPNER.

### 2. Library Construction

A healthy alpaca (1.5 years old, male, weighing approximately 80 kg, provided by Qingdao Kangda Biotechnology Co., Ltd.) was immunized. Multiple subcutaneous injections were administered in the neck and shoulder regions, and after five rounds of immunization, 100 mL of peripheral blood lymphocytes were collected from the alpaca. PBMCs were isolated using the Alpaca Peripheral Blood Lymphocyte Separation Kit from Tianjin Haoyang Biological. Total RNAs were extracted using the RNA Keeper Tissue Stabilizer Extraction Kit from Novozan. The extracted RNAs were reverse-transcribed into cDNAs using the HiScript III 1st Strand cDNA Synthesis Kit (Novozan, R312-01). Nested PCR was then employed to amplify the nucleic acid fragments of the variable region of the heavy-chain antibodies.

First-round of PCR:
Forward primer F1: GTCCTGGCTGCTCTTCTACAAGG (SEQ ID NO. 43);
Reverse primer R1: GGTACGTGCTGTTGAACTGTTCC (SEQ ID NO. 44);

The Fab fragment of the heavy chain antibody was amplified by PCR using cDNA as the template. The reaction program is as follows:

| Temperature | Time | Cycle |
|---|---|---|
| 95 °C | 5 min | - |
| 95 °C | 30 s | 15 cycles |
| 57 °C | 30 s | |
| 72 °C | 30 s | |
| 72 °C | 7 min | - |

Electrophoresis was performed using a 2% agarose gel. The band of approximately 700 bp was excised, and the target gene was recovered using a Gel Extraction Kit (Vazyme) according to the manufacturer's instructions.

### Second-round of PCR:

The first-round PCR product was used as the template to amplify the nanobody by a second PCR . The reaction system and procedure are shown in the table.
Forward primer F2: atggcccaggtgcagctgcagGAGTCTGGRGGAGG (SEQ ID NO. 45);
Reverse primer R2: gtggtgtgaggagacGGTGACCTGGGT (SEQ ID NO. 46);

The reaction system is as follows:

| Component | Volume |
|---|---|
| First-round PCR product | 1 µL |
| Forward primer F2(10µM) | 1 µL |
| Reverse primer R2(10µM) | 1 µL |
| 2× PCR Bestaq^{™} Master Mix | 25 µL |
| ddH₂O | Supplement to 50 µL |

The PCR reaction program is as follows:

| Temperature | Time | Cycle |
|---|---|---|
| 95 °C | 5 min | - |
| 95 °C | 30 s | 20 cycles |
| 55 °C | 30 s | |
| 72 °C | 30 s | |
| 72 °C | 5 min | - |

The nucleic acid fragments of the target heavy-chain single-domain antibody were recovered and cloned into the phage display vector pMES4 using the restriction enzymes Pst I and BstE II (purchased from NEB). Fresh competent cells of TG1 were prepared, and the products were electroporated into the TG1 competent cells. Library capacity was calculated to be 10¹¹ through gradient dilution and plating. Twenty clones were randomly selected for colony PCR and verified by sequencing, which showed an insertion rate of 100%. The construction of the heavy-chain single-domain antibody phage display library targeting PD-L1 was completed.

### 3. Panning for heavy chain single-domain antibodies targeting PD-L1.

10 µg/mL of PD-L1-his fusion protein (SEQ ID NO. 18) was coated on an immunotube, and incubated overnight at 4°C. The next day, the immunotube was blocked with 1 mL of PBS-milk (PBS containing 2% skim milk powder) at 37°C for 1 hour. At the same time, 100 µL of the phage library (derived from the aforementioned heavy-chain single-domain antibody phage display library) was blocked, and the blocked phage antibody library was added to the immunotube and incubated at 4°C overnight for binding. The liquid in the immunotube was discarded, then washed sequentially with PBS, PBST, and PBS + NaCl, and the washing stringency was increased with each round. After three rounds of washing, the liquid in the immunotube was discarded, 1 mL of 0.2 mol/L glycine-HCl (pH 2.2) was added for elution, and neutralized with 1 mol/L Tris to pH 7.4. E. coli TG1 at logarithmic phase was added to the immunotube for infection. The eluted and infected bacterial solution was resuspended, evenly spread onto a 2YT-AG plate (A: ampicillin sodium; G: glucose), and incubated at 37°C overnight. Then all colonies were collected. An appropriate amount of bacterial solution was transferred into 100 mL of 2YT-AG liquid medium, and cultured until the absorbance (A) at 600 nm reaches 0.7. Helper phage M13KO7 was added at a multiplicity of infection (MOI) of 50: 1, and incubated at 25°C for 30 minutes without shaking, then at 37°C and 150 rpm for 1 hour. IPTG was added to a final concentration of 0.15 mmol/L, and cultured at 30°C and 200 rpm for 10 hours. The phage antibodies were precipitated and recovered using PEG8000, and a certain amount was taken for the next round of screening. This process was repeated for a total of three rounds of selection. Ultimately, positive clones were enriched, thereby achieving the goal of screening for PD-L1-specific antibodies using phage display technology.

SEQ ID NO. 18:
MRIFAVFIFMTYWHLLNAFTVTVPKDLYVVEYGSNMTIECKFPVEKQLDLAALIVY
WEMEDKNIIQFVHGEEDLKVQHSSYRQRARLLKDQLSLGNAALQITDVKLQDAGVYRCM
ISYGGADYKRITVKVNAPYNKINQRILVVDPVTSEHELTCQAEGYPKAEVIWTSSDHQVLS
GKTTTTNSKREEKLFNVTSTLRINTTTNEIFYCTFRRLDPEENHTAELVIPELPLAHPPNERH
HHHHH.

### 4. Screen of positive clones using phage enzyme-linked immunosorbent assay (ELISA).

An obtained single colonies was picked from the panning output and cultured overnight in a 96-well plate containing 2×YT - AG medium at 37 °C and 220 rpm until saturation. The saturated bacterial solution was transferred so that the OD₆₀₀ was approximately 0.5, and the helper phage M13KO7 was added at an infection coefficient (MOI) of 50: 1, incubated at 25 °C for 30 min without shaking, and then cultured at 37 °C and 150 rpm for 1 h. Kanamycin was added to a final concentration of 20 µg/mL and IPTG was added to a final concentration of 0.15 mM, and induced at 30 °C and 200 rpm for 12 h. The supernatant, containing the phage antibodies, was collected by centrifugation. The target antigen (PDL1-his) and the control antigen (BSA) were coated at 4 °C overnight. The phage antibodies were allowed to bind at 37 °C for 1 h, washed, and then the anti-M13 antibody was allowed to bind at 37 °C for 45 min. After being washed, the TMB single-component color - developing solution was added, and the absorbance was measured at 450 nm. Clones were considered positive if the OD value in the sample well was more than three times that of the control well. Positive monoclonal colonies were sequenced to obtain the variable - region genes of 11 candidate antibodies.

### Example 2

This example provides a preliminary evaluation and identification of the heavy-chain single-domain antibody against PD-L1.

### 1. Expression and purification of the heavy-chain single-domain antibody in Escherichia coli.

The plasmids of the 11 single-domain antibodies (designated as PDL1-1, PDL1-3, PDL1-4, PDL1-6, PDL1-8, PDL1-13, PDL1-14, PDL1-16, PDL1-19, PDL1-20 and PDL1-21) obtained through sequencing analysis in Example 1 were extracted, and then transformed into BL21 (DE3) competent cells by heat shock. In a biosafety cabinet, 1 mL of positive clone bacterial solution was added to 100 mL of LB liquid medium (containing 100 µg/mL ampicillin), and shaken at 37 °C until the OD₆₀₀ reached approximately 0.8. IPTG was added to a final concentration of 1 mM, and incubated overnight with shaking at 30 °C. The next day, the bacteria were collected by centrifugation at 8000 rpm for 10 minutes, and the pellet was resuspended in 1.5 mL of pre-cooled TES buffer and stirred in an ice bath for 30 minutes. 3.0 mL of TES/4 (TES was diluted four-fold in distilled water) was added, and stirred in the ice bath for another 30 minutes. The supernatant (periplasmic protein extract) was collected by centrifugation at 9000 rpm for 10 minutes at 4 °C for SDS-PAGE analysis. Based on a protein purification system, affinity purification was performed using HisTrap^{™} FF, and the samples were eluted and collected with 300 mM imidazole buffer. The antibody concentration was quantified using a spectrophotometer, and the antibody purity was assessed by SDS-PAGE. The results are shown in Figure 2. The electrophoretic positions of the extracted proteins were consistent with expectations, with clear bands and high purity. No broken or degraded bands were observed for any of the PD-L1 proteins. Among them, lane 12 is the protein molecular weight marker, and lanes 1-11 are the purified nanobody protein samples of PDL1-1, PDL1-3, PDL1-4, PDL1-6, PDL1-8, PDL1-13, PDL1-14, PDL1-16, PDL1-19, PDL1-20, and PDL1-21, respectively.

### 2. Construction of antigen recombinant protein

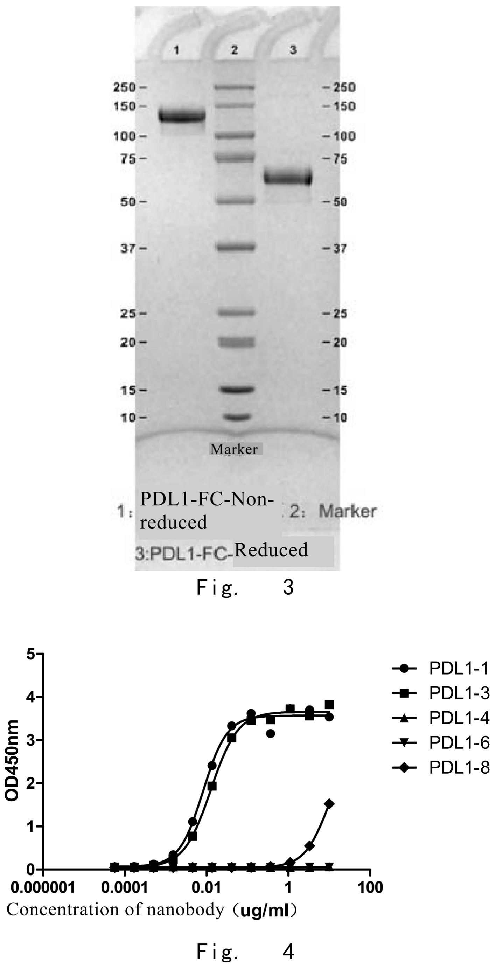

The expression vector for the recombinant protein of PD-L1 fused with human Fc (PD-L1-Fc) was constructed. The PD-L1 gene and human Fc gene were cloned into the pcDNA3.1 vector. After sequenced, the plasmid was transfected into healthy FreeStyle^{™} 293-F cells, which were cultured in a shaking flask at 37°C, 120 rpm, and 5% CO₂ on an orbital shaker for 3.5 days. The supernatant was collected and purified using a Protein A column, eluted with a citrate-sodium citrate buffer. Buffer exchange and concentration were carried out using an ultrafiltration concentrator tube. Protein purity was assessed by SDS-PAGE, and the results are shown in Figure 3: Lane 1, non-reduced SDS-PAGE of the PD-L1-Fc protein; Lane 2, protein molecular weight marker; Lane 3, reduced SDS-PAGE of the PD-L1-Fc protein. The electrophoretic position of the antigen recombinant protein was consistent with expectations, with clear bands and high purity. No broken or degraded bands of the PD-L1-Fc recombinant protein were observed. The protein concentration was quantified using a spectrophotometer, and the concentration was determined to be 2 mg/mL.

### 3. Detection of the binding of candidate PD-L1 nanobodies to human PD-L1 protein by ELISA.

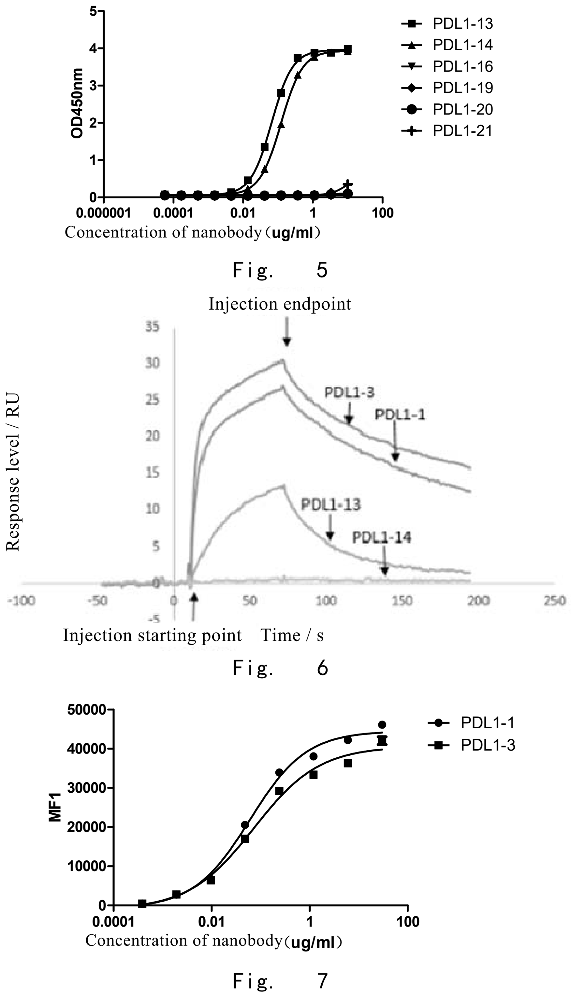

A plate was coated with 200 ng/well of human PDL1-Fc fusion protein (SEQ ID NO. 19) at 4°C overnight. Subsequently, the heavy chain single-domain antibodies obtained above was subjected to a 12-step 3-fold serial dilution starting from 10 µg/mL, blocked at 37°C for 1 hour. 100 µL/well of the blocked full-length antibody was added, incubated at 37°C for 1 hour and washed. And then 100 µL/well of HRP-conjugated anti-His tag antibody (His Tag Antibody HRP, Sino Biological) was added to the ELISA plate, incubated at 37°C for 45 minutes, and washed. 100 µL/well of TMB substrate solution for color development was added at 25°C for 10 minutes. 100 µL/well of 1 M H₂SO₄ was added to quench the reaction. The absorbance was measured at 450 nm using an enzyme-linked immunosorbent assay (ELISA) reader (Figures 4 and 5). Data processing and parameter fitting were performed using GraphPad Prism Software 5.0 to calculate the EC₅₀ values. The results are shown in Tables 1 and 2. Among the 11 screened nanobodies, 4 prokaryotically expressed nanobodies, PDL1-1, PDL1-3, PDL1-13, and PDL1-14 were found to bind to the PDL1-Fc fusion protein. Their corresponding amino acid sequences are shown in SEQ ID NOs: 13-16, respectively.

MRIFAVFIFMTYWHLLNAFTVTVPKDLYVVEYGSNMTIECKFPVEKQLDLAALIVYW
EMEDKNIIQFVHGEEDLKVQHSSYRQRARLLKDQLSLGNAALQITDVKLQDAGVYRCMIS
YGGADYKRITVKVNAPYNKINQRILVVDPVTSEHELTCQAEGYPKAEVIWTSSDHQVLSG
KTTTTNSKREEKLFNVTSTLRINTTTNEIFYCTFRRLDPEENHTAELVIPELPLAHPPNEREP
KSCDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK。

**Table 1**

| | PDL1-1 | PDL1-3 | PDL1-4 | PDL1-6 | PDL1-8 |
|---|---|---|---|---|---|
| EC50(ug/ml) | 0.008252 | 0.01278 | ~ 0.001032 | 1.821 | 9.787 |

**Table 2**

| | PDL1-13 | PDL1-14 | PDL1-16 | PDL1-19 | PDL1-20 | PDL1-21 |
|---|---|---|---|---|---|---|
| EC50(ug/ml) | 0.06611 | 0.1235 | 0.002282 | ~ 4.598 | 2.518 | 12.26 |

### Example 3

In this example, the SPR method (i.e., Biacore) was used to evaluate the binding ability of the PD-L1 single-domain antibody protein to PD-L1.

The single-concentration affinity screening assay was employed, in which all the nanobodies under investigation were tested at the same fixed concentration. This allowed for a parallel comparison of the tested nanobodies to assess differences in their binding activities. The capture antibody was immobilized on the surface of a CM5 chip using the Human Antibody Capture Kit. The kinetics parameters of the antibody-antigen interactions were determined using the multi-cycle kinetics method. Purified PDL1-His was diluted in HBS-EP buffer to a concentration of 1 µg/mL and captured on the chip surface at 25°C at a flow rate of 5 µL/min for 1 minute. The target response value (Response) was approximately 400 RU. The nanobodies PDL1-1, PDL1-3, PDL1-13, and PDL1-14 were diluted to 1.5 µg/mL in HBS-EP buffer as the running buffer. The assay conditions were set at 25°C and a flow rate of 30 µL/min, with an association time of 60 seconds and a dissociation time of 120 seconds. The regeneration condition involved 10 mM glycine-HCl buffer (pH 2.5) at a flow rate of 30 µL/min for 90 seconds. Results were analyzed and are shown in Figure 6 and Table 3. Antibody affinity is a key parameter for evaluating antibody molecules, and surface plasmon resonance (SPR) technology is widely recognized as the gold standard for measuring antibody affinity. Using the BIAcore T100 system and the single-concentration affinity screening assay, the binding activity parameters of PDL1-1, PDL1-3, and PDL1-13 were found to be 0.5, 0.6, and 0.2, respectively, while PDL1-14 showed no activity.

**Table 3**

| Sample | Binding value (60s) RU | Dissociation value (120s) RU | Dissociation percentage% | Binding activity evaluation parameter |
|---|---|---|---|---|
| PDL1-1 | 26.7 | -14.1 | 52.8 | 0.5 |
| PDL1-3 | 30.3 | -14.4 | 47.6 | 0.6 |
| PDL1-13 | 13.5 | -12 | 89.1 | 0.2 |

### Example 4

In this example, the FACS method was used to detect the binding activity of the nanobody to Raji-PDL1/Raji cells.

Raji cells stably transfected with the full-length human PD-L1 gene plasmid (Beijing Kangyuan Bochuang, KC-1886) was purchased and cultured to the logarithmic growth phase. The cells were resuspended in FACS buffer at a density of 3×10⁶/mL and aliquoted into a V-bottom plate at 100 µL per well. Then, the heavy-chain single-domain antibodies obtained in Example 2 (with the positive control group being KN035, Alphamab Oncology) as the primary antibody was subjected to a 5-fold serial dilution in FACS buffer to 30 µg/mL, added at 100 µL per well to resuspend the cells, incubated at 4°C for 60 minutes, and washed for three times with PBS. The cells were resuspended with the secondary antibody THE^{™} His Tag Antibody [FITC], monoclonal, mouse, incubated at 4°C for 60 minutes, and washed for three times with PBS. The cells were resuspended in 100-200 µL of PBS per well, and the MFI was detected using Beckman/CytoFLEX. Data processing and parameter fitting was performed using GraphPad Prism Software 5.0 to calculate the EC₅₀ values. Results are shown in Figure 7 and Table 4. PDL1-1 and PDL1-3 exhibited high binding activity to the PDL1 protein expressed on Raji cells and showed typical dose-dependent characteristics.

**Table 4**

| | PDL1-1 | PDL1-3 |
|---|---|---|
| EC₅₀ (ug/ml) | 0.0606 | 0.07298 |

### Example 5

In this embodiment, a competitive ELISA is employed to evaluate blocking effects of the PD-L1 Nanobody on the interaction between PD-1 and PD-L1.

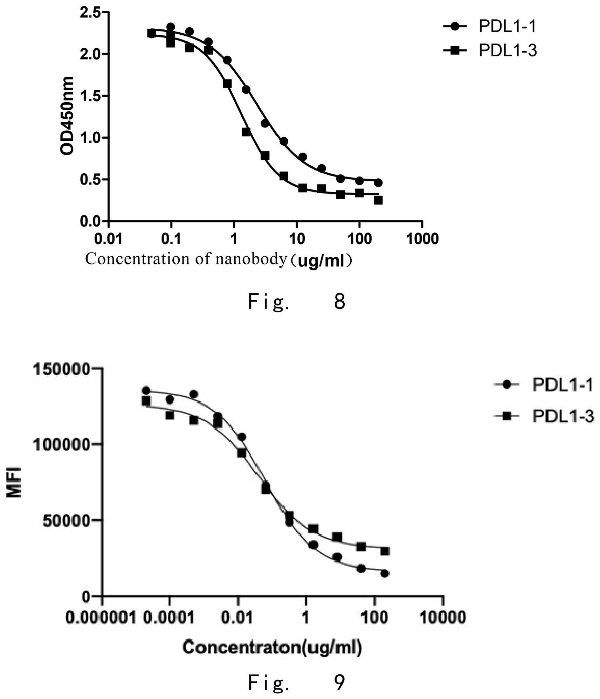

The PDL1-his protein was diluted in PBS to a concentration of 2 µg/mL, added at 100 µL per well to a 96-well ELISA plate, and incubated at 4°C overnight for coating. The VHHs obtained in Example 2 was subjected to a 2-fold serial dilution with 2.5% PBST-milk, starting from an initial concentration of 200 µg/mL, 13 dilution gradients, in which the final concentration of the recombinant PD1-Fc protein (SEQ ID NO. 20) was 50 µg/mL (the blank group receives no antibody or protein, only an equal volume of buffer), incubated at 37°C for 1 hour for blocking, then for another 1 hour at 37°C for binding, and washed. An anti-IgG antibody (purchased from Zhongshan Jinqiao) was added, incubated at 37°C for 45 minutes, and washed again. Then 100 µL of TMB substrate was added per well for color development, incubated at 25°C for 10 minutes, and quenched by adding 100 µL of 1 M H₂SO₄ per well. The absorbance was measured at 450 nm using a microplate reader. Data processing and parameter fitting were performed using GraphPad Prism Software 5.0 to calculate the half-maximal inhibitory concentration (IC₅₀). The results are shown in Figure 8 and Table 5, indicating that PDL1-1 and PDL1-3 can block the binding of PDL1-his protein to PD1-Fc within a certain concentration range in a dose-dependent manner. The IC₅₀ values of PDL1-1 and PDL1-3 are 2.323 µg/mL and 1.312 µg/mL, respectively, showing similar blocking activities.

**Table 5**

| | PDL1-1 | PDL1-3 |
|---|---|---|
| IC₅₀ (ug/ml) | 2.323 | 1.312 |

SEQ ID NO. 20:
PGWFLDSPDRPWNPPTFSPALLVVTEGDNATFTCSFSNTSESFVLNWYRMSPSNQTD
KLAAFPEDRSQPGQDCRFRVTQLPNGRDFHMSVVRARRNDSGTYLCGAISLAPKAQIKES
LRAELRVTERRAEVPTAHPSPSPRPAGQFQDKTHTCPPCPAPELLGGPSVFLFPPKPKDTLM
ISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSCSVMHEA
LHNHYTQKSLSLSPGK。

### Example 6

In this example, the FACS method was used to detect the blocking activity of antibodies at the cellular level (cell-based RBA).

The Raji cells stably transfected with the full-length human PD-L1 gene plasmid were cultured to the logarithmic growth phase, resuspended in FACS buffer, and adjusted to a cell density of 3×10⁶ cells/mL, and plated at 100 µL per well. Then, 50 µL/well of PD1-Fc and 50 µL/well of the antibody to be tested were added, and the cells were resuspended, incubated at 4°C for 60 minutes, washed for three times in 200 µL/well of PBS, and centrifuged at 300 g for 5 minutes. The supernatant was discarded. The secondary antibody SA-488 was diluted in FACS buffer, and 100 µL/well was used to resuspend the cells, followed by incubation at 4°C for 60 minutes. The cells were washed for three times with 200 µL/well of PBS, centrifuged at 300 g for 5 minutes, and the supernatant was discarded. The cells were resuspended in 100 µL/well of PBS and analyzed by flow cytometry. Results are shown in Figure 9 and Table 6, demonstrating that PDL1-1 and PDL1-3 could block the binding of PD-L1 protein on the surface of Raji cells to PD1-Fc within a certain concentration range in a dose-dependent manner. The IC₅₀ values of PDL1-1 and PDL1-3 were 0.06263 µg/mL and 0.04058 µg/mL, respectively, indicating that their blocking activities were similar.

**Table 6**

| | PDL1-1 | PDL1-3 |
|---|---|---|
| IC50(ug/ml) | 0.06263 | 0.04058 |

### Example 7

In this example, the PD-L1 nanobody was analyzed for the binding specificity to the PDL1-Fc protein.

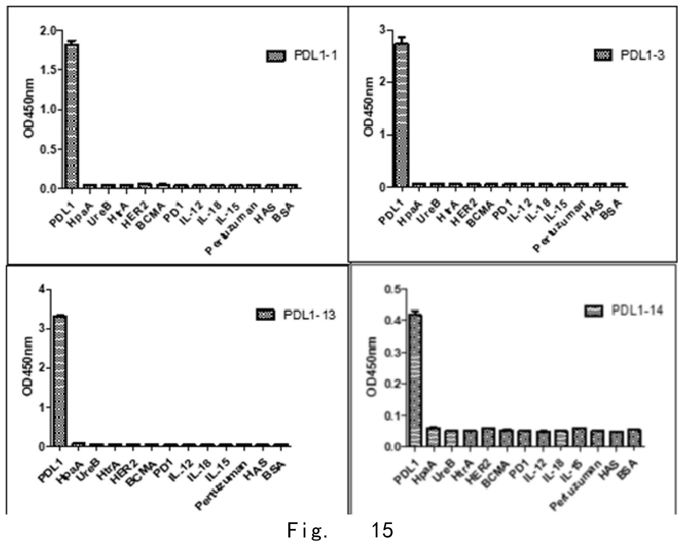

Different proteins, including antigens HpaA, UreB, HtrA, HER2, BCMA, PD1, IL-12, IL-18, IL-15, Pertuzumab, HAS, BSA, and PD-L1 (prepared in-house), were coated in the wells at 200 ng/well at 4°C overnight. Antibodies PDL1-1 (0.2 µg), PDL1-3 (0.2 µg), PDL1-13 (0.2 µg), and PDL1-14 (0.2 µg) were added in duplicate for detection. After incubated at 37°C for 1 hour, the plates were washed, followed by the addition of HRP-conjugated anti-His tag antibody at 100 µL per well into the ELISA plate. The plates were then incubated at 37°C for 45 minutes, and washed. TMB substrate solution was added at 100 µL per well for color development, and the reaction was quenched by adding 100 µL of 1M H₂SO₄ per well. The absorbance was measured at 450 nm wavelength using an enzyme-linked immunosorbent assay (ELISA) reader. Data was processed using GraphPad Prism Software 5.0. The results of the specificity experiment shown in Figure 15 indicate that PDL1-1, PDL1-3, PDL1-13, and PDL1-14 specifically bind only to human PDL1 and do not bind to any of the other unrelated proteins.

### Example 8

In this example, mammalian cells were used to prepare the NbsPDL1-Fc fusion protein.

The clones identified as positive single-chain antibodies (PDL1-1, PDL1-3, and PDL1-13) were sequenced, and the sequencing results were analyzed. The positive single-chain variable region genes were amplified using primers HF (ccttaagggcgtgcagtgccaggtgcagctgcaggagtc; SEQ ID NO. 47) and HR (gatttgggctcgctagctgaggagacggtgacctggg; SEQ ID NO. 48). The amplification reaction system is shown in Table 7, and the reaction procedure is shown in Table 8. The positive single-chain antibody genes were cloned into the pcDNA3.1(+) vector harboring human Fc (SEQ ID NO. 21) via homologous recombination. The reaction system is shown in Table 9, and the reaction conditions were 37°C for 30 minutes.

SEQ ID NO. 21:
EPKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPGK。

**Table 7**

| | |
|---|---|
| Phagemid template | 0.5 µL |
| HF primer (10µM) | 1 µL |
| HR primer (10µM) | 1 µL |
| 2×Phanta Max Master Mix | 15 µL |
| Sterilized deionized water | 12.5 µL |
| Sum | 30 µL |

**Table 8**

| Temperature | Time | Cycle |
|---|---|---|
| 95°C | 3min | - |
| 95°C | 15s | 30 cycles |
| 62°C | 15s | |
| 72°C | 20s | - |
| 72°C | 5min | - |
| 4°C | maintain | - |

**Table 9**

| | |
|---|---|
| Vector pABG1-Fc(0.02 pmol) | 0.4 µL |
| PD-L1 target fragment (0.06 pmol) | 0.3~3 µL |
| Exnase II | 1 µL |
| 5×CE II buffer | 2 µL |
| Sterilized deionized water | 3.6~6.3 µL |
| Sum | 10 µL |

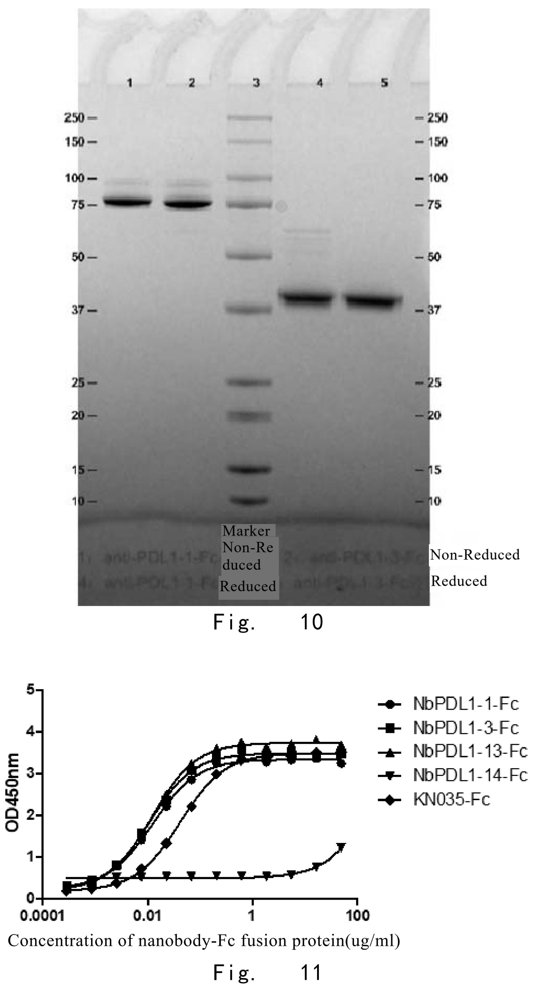

Plasmids were extracted using an endotoxin-free plasmid extraction kit, quantified with a spectrophotometer, and sequenced. One day before transfection, FreeStyle^{™} 293-F cells were collected by centrifugation, and adjusted to a cell density of 1 × 10⁶ cells/mL. The transfection ratio was as follows: (cells : medium = 20 : 1); Polyethylenimine Linear (PEI) at 2 µL/mL; plasmid at 1 µg/mL. That is, 40 µg of plasmid was dissolved in 2 mL of Opti-MEM medium, gently mixed, followed by the addition of 80 µL of PEI transfection reagent. The mixture was vortexed for 10 seconds to ensure thorough mixing, then incubated at 25°C for 13 minutes. 2 mL of the resulting mixture was added to 40 mL of healthy FreeStyle^{™} 293-F cells, gently mixed, and cultured in a shaking flask at 37°C, 120 rpm, and 5% CO₂ on a horizontal shaker for 4 days. The supernatant was collected for SDS-PAGE analysis. Based on a protein purification system, Protein A affinity purification was employed, and samples were eluted and collected using 0.1 M citrate-sodium citrate buffer. The antibody concentration was quantified using a spectrophotometer, and the purity of the antibodies was assessed by SDS-PAGE, as shown in Figure 10. Both the reduced and non-reduced forms of the NbPDL1-Fc fusion proteins migrated to the expected positions, with clear bands and high purity. No broken or degraded bands were observed for any of the NbPDL1-Fc fusion proteins. Among them, lanes 1 and 2 are the non-reduced form of the NbPDL1-1-Fe fusion protein, lane 3 is the protein molecular weight marker, and lanes 4 and 5 are the reduced forms of the NbPDL1-1-Fc fusion protein and the NbPDL1-3-Fc fusion protein, respectively.

### Example 9

In this example, the binding of the candidate NbsPD-L1-Fc fusion protein to human PD-L1 protein was detected.

The plate was coated with 200 ng/well of human PDL1-his protein and incubated overnight at 4°C. Subsequently, the heavy chain single-domain antibodies obtained in Example 7 (with the positive control group being the Fc-containing KN035 (SEQ ID NO. 22) from CanSino Biologics) were subjected to a serial 3-fold dilution, 12 gradients starting from 10 µg/mL. After being blocked at 37°C for 1 hour, 100 µL/well of the blocked full-length antibodies were added to the plate and incubated at 37°C for 1 hour, and washed. Then, HRP-conjugated anti-his tag antibody was added at 100 µL/well to the ELISA plate, incubated at 37°C for 45 minutes, and washed. TMB substrate solution was added at 100 µL/well and incubated at 25°C for 10 minutes. The reaction was then quenched by adding 1 M H₂SO₄ at 100 µL/well. The absorbance was measured at 450 nm using a microplate reader. Data processing and parameter fitting were performed using GraphPad Prism Software 5.0 to calculate the EC₅₀ values. The results, as shown in Table 10 and Figure 11, indicate that the NbPDL1-1-Fc fusion protein, NbPDL1-3-Fc fusion protein, and NbPDL1-13-Fc fusion protein exhibited better binding activity compared with the control antibody Fc-containing KN035, and displayed typical dose-dependent characteristics.

**Table 10**

| | NbPDL1-1-Fc | NbPDL1-3-Fc | NbPDL1-13-Fc | KN035-Fc |
|---|---|---|---|---|
| EC₅₀ (ug/ml) | 0.01237 | 0.01106 | 0.01212 | 0.04186 |

SEQ ID NO. 22:
QVQLVESGGGLVQPGGSLRLSCAASGKMSSRRCMAWFRQAPGKERERVAKLLTTS
GSTYLADSVKGRFTISRDNSKNTVYLQMNSLRAEDTAVYYCAADSFEDPTCTLVTSSGAF
QYWGQGTLVTVSSEPKSSDKTHTCPPCPAPELLGGPSVFLFPPKPKDTLMISRTPEVTCVV
VAVSHEDPEVKFNWYVDGVEVHNAKTKPREEQYNSTYRVVSVLTVLHQDWLNGKEYK
CKVSNKALPAGIEKTISKAKGQPREPQVYTLPPSRDELTKNQVSLTCLVKGFYPSDIAVEW
ESNGQPENNYKTTPPVLDSDGSFFLYSKLTVDKSRWQQGNVFSCSVMHEALHNHYTQKS
LSLSPGK。

### Example 10

In this example, the binding ability of the candidate NbPD-L1-Fc fusion protein to the PD-L1 (Cynomolgus) protein was detected.

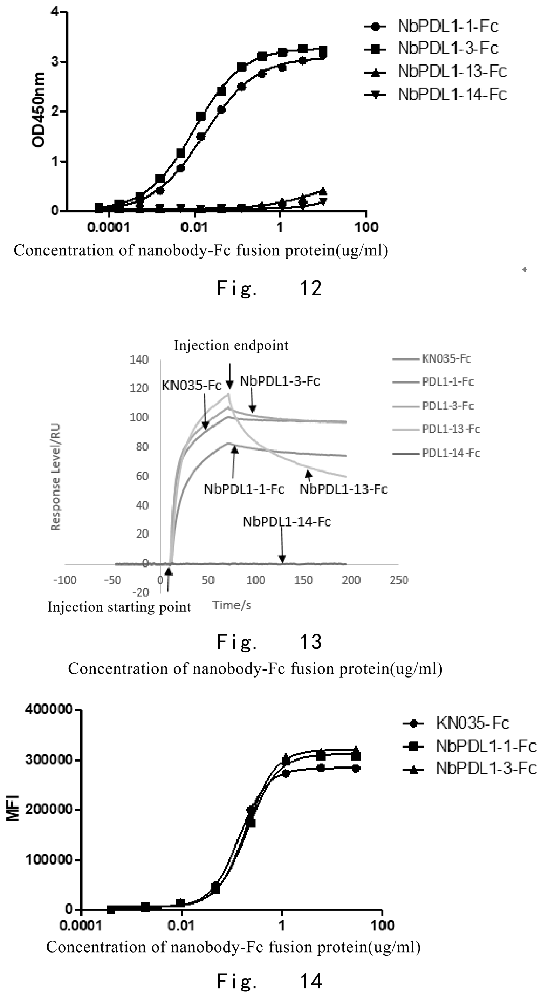

A plate was coated with 100 ng/well of Cynomolgus PDL1-His protein at 4 °C overnight. The coating solution was discarded, and 120 µL of 2.5% PBS-milk (PBS containing 2.5% skim milk powder) was added to each well. The heavy-chain single-domain recombinant antibodies obtained in Example 7 (with the positive control being KN035, Alphamab Oncology) were serially diluted 3-fold in 2.5% PBST across 12 gradients starting from 50 µg/mL. After blocking at 37 °C for 1 h, the blocking solution was discarded, and 100 µL/well of the blocked full antibodies were added, and incubated at 37 °C for 1 h. The plate was washed for six times with PBST. The HRP-conjugated goat anti-human IgG antibody was diluted at 1:3000 in 2.5% PBST-milk, and added at 100 µL/well to the plate. The plate was incubated at 37 °C for 45 min, washed for six times with PBST, and the washing solution was discarded. 100 µL/well of TMB substrate solution was added, and incubated at 25 °C for 10 min. The reaction was quenched by adding 100 µL/well of 1 M H₂SO₄. The absorbance was measured at 450 nm using a microplate reader. The results are shown in Figure 12 and Table 11. NbPDL1-1-Fc and NbPDL1-3-Fc can effectively bind to the Cynomolgus PDL1 protein in a dose-dependent manner.

**Table 11**

| | NbPDL1-1-Fc | NbPDL1-3-Fc | NbPDL1-13-Fc | NbPDL1-14-Fc |
|---|---|---|---|---|
| EC₅₀ (ug/ml) | 0.01625 | 0.009659 | 5.099 | 31.88 |

### Example 11

In this example, the SPR method (i.e., Biacore) was used to evaluate the binding ability of the PD-L1 single-domain antibody Fc fusion protein to PD-L1.

All the nanobodies to be tested were evaluated at the same single concentration by using the single-concentration affinity screening assay method. The nanobodies were compared in parallel for the difference in the binding activities. The capture antibody was coupled to the surface of a CM5 chip using the Human Antibody Capture Kit, and the kinetic parameters of the antibody-antigen interaction were determined using the multi-cycle kinetics method. The purified PDL1-His protein was diluted with HBS-EP buffer to a concentration of 1 µg/mL and captured on the chip surface under conditions of 25°C and 5 µL/min for 1 minute, achieving a target response value (Response) of approximately 400 RU. The nanobodies PDL1-1-Fc, PDL1-3-Fc, PDL1-13-Fc, and PDL1-14-Fc were diluted with HBS-EP buffer to a concentration of 1.5 µg/mL and used as the running buffer. The testing conditions were set at 25°C and a flow rate of 30 µL/min, with a 60-second association phase and a 120-second dissociation phase. The regeneration condition was set at 10 mM glycine-HCl buffer (pH 2.5) at a flow rate of 30 µL/min for 90 seconds; and the results were analyzed.

As shown in Table 12 and Figure 13, NbPDL1-1-Fc, NbPDL1-3-Fc, and NbPDL1-13-Fc can effectively bind to PD-L1, with binding activity parameters of 8.2, 12.7, and 2.4, respectively. NbPDL1-14 exhibits no binding activity.

**Table 12**

| Sample | Binding value (60s)RU | Dissociation value (120s)RU | Dissociation percentage% | Parameter for evaluating binding activity |
|---|---|---|---|---|
| NbPDL1-1-Fc | 83.1 | -8.4 | 10.1 | 8.2 |
| NbPDL1-3-Fc | 105.7 | -8.8 | 8.3 | 12.7 |
| NbPDL1-13-Fc | 112.3 | -51.8 | 46.1 | 2.4 |

### Example 12

In this example, the FACS method was used to detect the binding activity of the NbPDL1-1-Fc fusion protein to Raji-PDL1/Raji cells.

Raji cells harboring the full-length human PDL1 gene plasmid (Beijing Kangyuan Bochuang, KC-1886) were purchased and cultured to the logarithmic growth phase. The cells were resuspended in FACS buffer, adjusted to a cell density of 3×10⁶/mL, and plated at 100 µL per well. Subsequently, the heavy chain single-domain antibodies obtained in Example 7 (the positive control group used is Fc-containing KN035, Alphamab Oncology) as the primary antibody was subjected to a 5-fold dilution with FACS buffer to a concentration of 30 µg/mL, added at 100 µL per well to resuspend the cells and incubated at 4°C for 60 minutes. The plate was washed for three times with PBS, then the secondary antibody THE^{™} His Tag Antibody [FITC], monoclonal, mouse was added to resuspend the cells and incubated at 4°C for 60 minutes. The plate was washed for three times with PBS, and 100 µL of PBS per well was added to resuspend the cells, and Beckman/CytoFLEX was used to detect the mean fluorescence intensity (MFI). Data processing and parameter fitting was performed by using GraphPad Prism Software 5.0 to calculate the EC₅₀ values. The results are shown in Table 13 and Figure 14. The affinities of the NbPDL1-1-Fc fusion protein and the NbPDL1-3-Fc fusion protein are comparable to that of the Fc-containing KN035.

**Table 13**

| | KN035-Fc | NbPDL1-1-Fc | NbPDL1-3-Fc |
|---|---|---|---|
| EC₅₀ (ug/ml) | 0.1392 | 0.2041 | 0.1968 |

### Example 13

In this example, the PDL1-1 single-domain antibody was humanized.

The protein surface amino acid humanization method was used. A reference homologous sequence of fully human origin is aligned with sequences in IMGT, and based on design principles, the surface amino acids of the protein are humanized by introducing site-directed mutagenesis to complete the antibody humanization.

20 humanized antibody strains were obtained through the humanization of the PDL1-1 antibody.

### Humanized sequence

PDL1-1v1 
EVQLLESGGGLVQPGGSLRLSCAASGFTVSNSAVSWARQAPGKGLEWVSLIDSNRNT
VYAQSVRGRFTISRDNSKNTVYLQMNSLRAEDTAVYYCAKGIWGSTLWGQGTTVTVSS
(SEQ ID NO. 23).
PDL1-1v2 
QVQLVESGGGLVKPGGSLRLSCAASGFTVSNSAVSWARQAPGKGLEWVSLIDSNRN
TVYAQSVRGRFTISRDNAKNSVYLQMNSLRAEDTAVYYCAKGIWGSTLWGQGTTVTVSS
(SEQ ID NO. 24).
PDL1-1v3 
QVQLLESGGGLVQPGGSLRLSCAASGFTVSNSAVSWARQAPGKGLEWVSLIDSNRN
TVYAQSVRGRFTISRDNSKNTVYLQMNSLRAEDTAVYYCAKGIWGSTLWGQGTTVTVSS
(SEQ ID NO. 25).
PDL1-1v4 
EVQLQESGGGLVQPGGSLRLSCAASGFTVSNSAVSWARQAPGKGLEWVSLIDSNRN
TVYAQSVRGRFTISRDNSKNTVYLQMNSLRAEDTAVYYCAKGIWGSTLWGQGTTVTVSS
(SEQ ID NO. 26).
PDL1-1v5 
EVQLLESGGGLVQPGGSLRLSCAASGFTVSNSAVSWARQAPGKGLEWVSLIDSNRNT
VYAQSVRGRFTISRDNVKNTVYLQMNSLRAEDTAVYYCAKGWGSTLWGQGTTVTVSS
(SEQ ID NO. 27).
PDL1-1v6 
EVQLLESGGGLVQPGGSLRLSCAASGFTVSNSAVSWARQAPGKGLEWVSLIDSNRNT
VYAQSVRGRFTISRDNSKNTVYLQMNSLKAEDTAVYYCAKGIWGSTLWGQGTTVTVSS
(SEQ ID NO. 28).
PDL1-1v7 
EVQLLESGGGLVQPGGSLRLSCAASGFTVSNSAVSWARQAPGKGLEWVSLIDSNRNT
VYAQSVRGRFTISRDNSKNTVYLQMNSLRPEDTAVYYCAKGIWGSTLWGQGTTVTVSS
(SEQ ID NO. 29).
PDL1-1v8 
EVQLLESGGGLVQPGGSLRLSCAASGFTVSNSAVSWARQAPGKGLEWVSLIDSNRNT
VYAQSVRGRFTISRDNSKNTVYLQMNSLRAEDTAVYYCAKGIWGSTLTGQGTTVTVSS
(SEQ ID NO. 30).
PDL1-1v9 
EVQLLESGGGLVQPGGSLRLSCAASGFTVSNSAVSWARQAPGKGLEWVSLIDSNRNT
VYAQSVRGRFTISRDNSKNTVYLQMNSLRAEDTAVYYCAKGIWGSTLWGQGTQVTVSS
(SEQ ID NO. 31).
PDL1-1v10- 
EVQLLESGGGLVQPGGSLRLSCAASGFTVSNSAVSWARQAPGKGLEWVSLIASNRNT
VYAQSVRGRFTISRDNSKNTVYLQMNSLRAEDTAVYYCAKGIWGSTLWGQGTTVTVSS
(SEQ ID NO. 32).
PDL1-1v11 
QVQLQESGGGLVKPGGSLRLSCAASGFTVSNSAVSWARQAPGKGLEWVSLIDSNRN
TVYAQSVRGRFTISRDNAKNSVYLQMNSLRAEDTAVYYCAKGIWGSTLWGQGTTVTVSS
(SEQ ID NO. 33).
PDL1-1v12 
QVQLVESGGGLVQPGGSLRLSCAASGFTVSNSAVSWARQAPGKGLEWVSLIDSNRN
TVYAQSVRGRFTISRDNAKNSVYLQMNSLRAEDTAVYYCAKGIWGSTLWGQGTTVTVSS
(SEQ ID NO. 34).
PDL1-1v13 
QVQLVESGGGLVKPGGSLTLSCAASGFTVSNSAVSWARQAPGKGLEWVSLIDSNRNT
VYAQSVRGRFTISRDNAKNSVYLQMNSLRAEDTAVYYCAKGIWGSTLWGQGTTVTVSS
(SEQ ID NO. 35).
PDL1-1v14 
QVQLVESGGGLVKPGGSLRLSCAASGFTVSNSAVSWARQAPGKGLEWVSLIDSNRN
TVYAQSVRGRFTISRDNVKNSVYLQMNSLRAEDTAVYYCAKGIWGSTLWGQGTTVTVSS
(SEQ ID NO. 36).
PDL1-1v15 
QVQLVESGGGLVKPGGSLRLSCAASGFTVSNSAVSWARQAPGKGLEWVSLIDSNRN
TVYAQSVRGRFTISRDNAKNTVYLQMNSLRAEDTAVYYCAKGIWGSTLWGQGTTVTVSS
(SEQ ID NO. 37).
PDL1-1v16 
QVQLVESGGGLVKPGGSLRLSCAASGFTVSNSAVSWARQAPGKGLEWVSLIDSNRN
TVYAQSVRGRFTISRDNAKNSVYLQMNSLKAEDTAVYYCAKGIWGSTLWGQGTTVTVSS
(SEQ ID NO. 38).
PDL1-1v17 
QVQLVESGGGLVKPGGSLRLSCAASGFTVSNSAVSWARQAPGKGLEWVSLIDSNRN
TVYAQSVRGRFTISRDNAKNSVYLQMNSLRPEDTAVYYCAKGIWGSTLWGQGTTVTVSS
(SEQ ID NO. 39).
PDL1-1v18 
QVQLVESGGGLVKPGGSLRLSCAASGFTVSNSAVSWARQAPGKGLEWVSLIDSNRN
TVYAQSVRGRFTISRDNAKNSVYLQMNSLRAEDTAVYYCAKGIWGSTLTGQGTTVTVSS
(SEQ ID NO. 40).
PDL1-1v19 
QVQLVESGGGLVKPGGSLRLSCAASGFTVSNSAVSWARQAPGKGLEWVSLIDSNRN
TVYAQSVRGRFTISRDNAKNSVYLQMNSLRAEDTAVYYCAKGIWGSTLWGQGTQVTVSS
(SEQ ID NO. 41).
PDL1-1v20

QVQLVESGGGLVKPGGSLRLSCAASGFTVSNSAVSWARQAPGKGLEWVSLIASNRN
TVYAQSVRGRFTISRDNAKNSVYLQMNSLRAEDTAVYYCAKGIWGSTLWGQGTTVTVSS
(SEQ ID NO. 42).

### Example 14

In this example, the function of the PD-L1-1 humanized single-domain antibody was identified.

### 14.1 Preparation of humanized single-domain antibody against protein PDL1-1 using mammalian cells

The sequence gene of the humanized single-domain antibody PDL1-1 was transferred into the vector pcDNA3.1 to transfect FreeStyle^{™} 293-F cells. The cell density was adjusted to 1 × 10⁶ cells/mL with a transfection ratio (cells : medium = 20 : 1). Polyethylenimine Linear (PEI) at 2 µL/mL, plasmid DNA at 1 µg/mL; and 80 µL of PEI transfection reagent were incubated at 25°C for 10 minutes; added to the FreeStyle^{™} 293-F cells, and cultured in a horizontal shaker flask at 37°C, 120 rpm, and 5% CO₂ for 3.5 days. Based on the protein purification system, HisTrap^{™} FF was used for affinity purification, and the sample was eluted and collected with 300 mM imidazole buffer. The supernatant was collected for SDS-PAGE electrophoresis analysis.

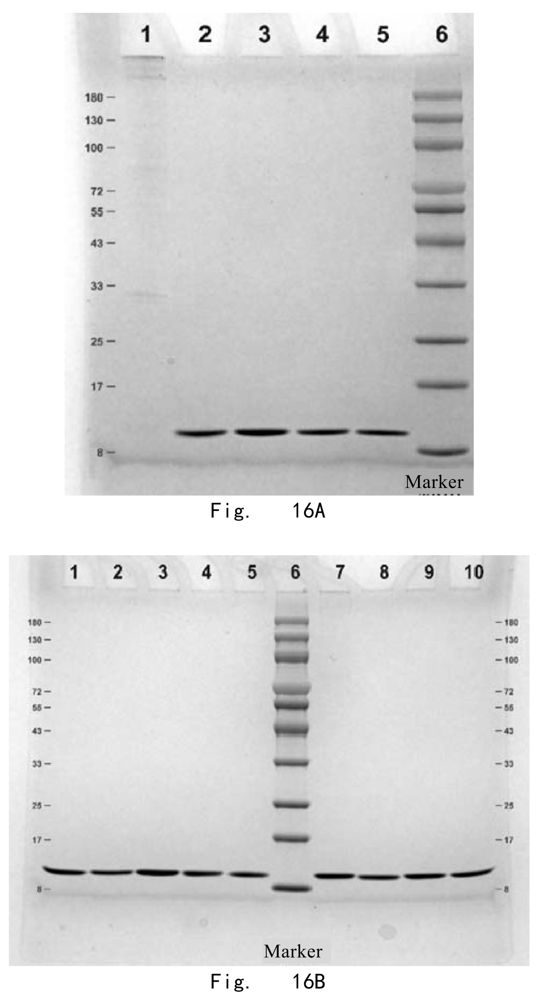

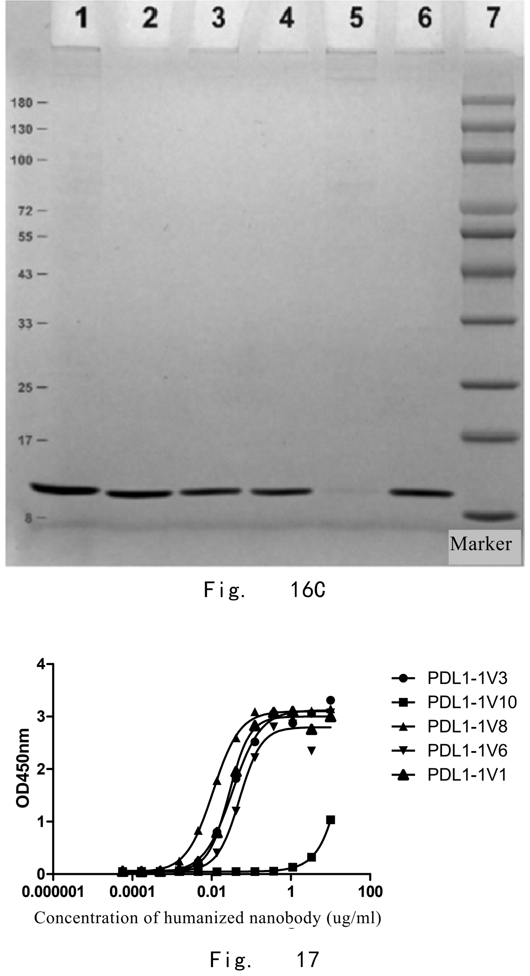

The results are shown in Figures 16A-16C, in which non-reduced SDS-PAGE gel electrophoresis was used to characterize the PDL1-1 humanized single-domain antibodies. (Figure 16A): 1: PDL1-1v4, 2: PDL1-1v7, 3: PDL1-1v9, 4: PDL1-1v1, 5: PDL1-1v2, 6: Protein molecular weight marker; (Figure 16B): 1: PDL1-1v3, 2: PDL1-1v10, 3: PDL1-1v8, 4: PDL1-1v5, 5: PDL1-1v6, 6: Protein molecular weight marker, 7: PDL1-1v20, 8: PDL1-1v13, 9: PDL1-1v19, 10: PDL1-1v17; (Figure 16C): 1: PDL1-1v11, 2: PDL1-1v14, 3: PDL1-1v16, 4: PDL1-1v15, 5: PDL1-1v12, 6: PDL1-1v18, 7: Protein molecular weight marker. The results indicate that the electrophoretic positions of the humanized single-domain antibody proteins are consistent with expectations, with clear bands and relatively high purity. No broken or degraded bands were observed.

### 14.2 Identification of the binding ability of the humanized single-domain antibody PD-L1-1 to PD-L1 by ELISA

The human PDL1-Fc recombinant protein was coated onto a plate at 200 ng per well and incubated overnight at 4°C. Subsequently, 20 humanized single-domain antibodies obtained from 14.1 were serially diluted 3-fold across 12 concentration gradients starting from 10 µg/mL. The plate was blocked at 37°C for 1 h, and incubated with the antibodies at 37°C for 1 h. After washing, HRP-conjugated anti-His tag antibody (His Tag Antibody HRP, Sino Biological) was added, and incubated at 37°C for 45 min. After washing, TMB substrate solution was added for color development, and the reaction was quenched with 1 M H₂SO₄. The absorbance at 450 nm was measured using a microplate reader. Data processing and parameter fitting were performed using GraphPad Prism Software 5.0 to calculate the EC₅₀ values (Tables 14-17).

**Table 14**

| | PDL1-1V3 | PDL1-1V10 | PDL1-1V8 | PDL1-1V6 | PDL1-1V1 |
|---|---|---|---|---|---|
| EC₅₀ (ug/ml) | 0.03357 | ~ 109.7 | 0.01084 | 0.05046 | 0.02747 |

**Table 15**

| | PDL1-1V4 | PDL1-1V5 | PDL1-1V7 | PDL1-1V9 | PDL1-1V1 |
|---|---|---|---|---|---|
| EC₅₀ (ug/ml) | 3.997 | 0.04781 | 0.028 | 0.02596 | 0.02508 |

**Table 16**

| | PDL1-1V11 | PDL1-1V14 | PDL1-1V16 | PDL1-1V15 | PDL1-1V2 |
|---|---|---|---|---|---|
| EC50(ug/ml) | 0.02 | 0.0342 | 0.06285 | 0.04318 | 0.04228 |

**Table 17**

| | PDL1-1 V12 | PDL1-1V 13 | PDL1-1V 20 | PDL1-1 V17 | PDL1-1 V18 | PDL1-1 V19 | PDL1-1V2 |
|---|---|---|---|---|---|---|---|
| EC₅₀ (ug/ml) | 1.244 | 0.04608 | 0.8745 | 0.06636 | 0.01819 | 0.04843 | 0.0590 9 |

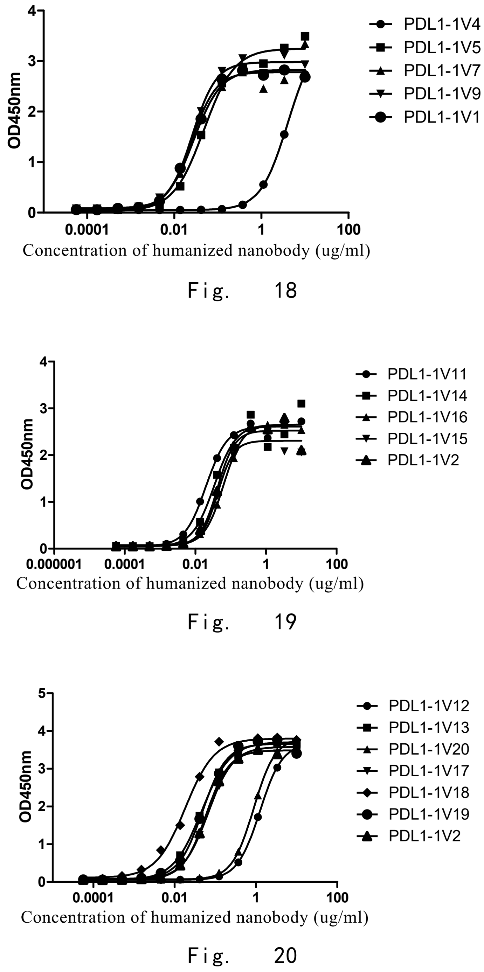

The binding activity of 20 purified humanized single-domain antibodies (PD-L1-1) to PDL1-Fc protein was analyzed by ELISA. The results (Figures 17-20) showed that the PD-L1-1 humanized single-domain antibodies exhibited high binding activity to the PDL1-Fc protein, and the binding displayed a typical dose-dependent characteristic.

### 14.3 ForteBIOTM system was used to measure the changes in affinity between each mutant and the parental antibody.

ForteBIO^{™} Octet QK^{e} biomolecular interaction real-time detection system is an eight-channel detection platform based on Bio-Layer Interferometry (BLI). Based on the high-throughput characteristics of this platform, a comparative analysis of the kinetic profiles of interactions between different mutant antibody molecules and various mutant antigens were conducted. The specific method is as follows:
1) the parental PDL1-Fc and each mutant was diluted to 100 nM separately in 10 mM PBS + 0.05% Tween-20 buffer, and added at 200 µL per well into a 96-well plate for detection;
2) the Loading > Baseline > Association > Dissociation procedure was conducted, the Association phase was set to 2 minutes and the Dissociation phase to 3 minutes. Both the Baseline and Dissociation processes were carried out in 10 mM PBS + 0.05% Tween-20 buffer, the Loading phase corresponded to PDL1-Fc dilution at a specific concentration, and the Association phase corresponded to the mutant dilution at a specific concentration;
3) the results were fit and analyzed by Data Analysis to determine the affinity constant K_{D} of different PDL1-1 mutants.

The results are shown in Table 18, wherein KD: equilibrium dissociation constant.

**Table 18**

| Sample ID | K_{D}(M) |
|---|---|
| 1v1 | 1.313E-08 |
| 1v2 | 1.428E-08 |
| 1v3 | 1.85E-08 |
| 1v4 | 1.316E-06 |
| 1v5 | 1.22E-08 |
| 1v6 | 2.205E-08 |
| 1v7 | 1.09E-08 |
| 1v8 | 5.901E-09 |
| 1v9 | 1.210E-08 |
| 1v10 | 3.05E-08 |
| 1v11 | 5.86E-09 |
| 1v12 | 3.890E-07 |
| 1v13 | 1.136E-08 |
| 1v14 | 7.37E-09 |
| 1v15 | 1.08E-08 |
| 1v16 | 1.43E-08 |
| 1v17 | 1.017E-08 |
| 1v18 | 4.158E-09 |
| 1v19 | 9.859E-09 |
| 1v20 | 3.25E-08 |

The affinity constants of the recombinant proteins PDL1-Fc and PDL1-1 mutants were determined using a capture assay. The measured affinity constant results are shown in Table 18. The affinity of mutant 1v8 for PDL1-Fc was 5.901 nM, the affinity of mutant 1v11 was 5.86 nM, the affinity of mutant 1v14 was 7.37 nM, the affinity of mutant 1v18 was 4.158 nM, and the affinity of mutant 1v19 was 9.859 nM, which was significantly higher than that of the other mutants.

### 14.4 Elisa assay to evaluate the blocking effect of the humanized heavy-chain single-domain antibody PD-L1-1 on the interaction between PD-1 and PD-L1

The PDL1-his protein was diluted to 2 µg/mL in PBS and coated overnight at 4°C. The humanized heavy-chain single-domain antibody obtained in 14.1 was subjected to a 2-fold serial dilution in a 2.5% PBST-milk solution starting from an initial concentration of 200 µg/mL across 13 dilution gradients, including PD1-Fc (SEQ ID NO. 20) recombinant protein at a final concentration of 50 µg/mL (the blank group receives no antibody or protein, only an equal volume of buffer), blocked at 37°C for 1 hour, then incubated at 37°C for 1 hour for binding. After washing, an anti-IgG antibody (purchased from Zhongshan Jinqiao) was added, incubated at 37°C for 45 minutes, and washed again, and then TMB substrate color-developing solution was added. The mixture was incubated at 25°C for 10 minutes, quenched by 100 µL of 1 M H₂SO₄ per well, and the absorbance at 450 nm was measured using a microplate reader. The data were processed and the parameters were fit using GraphPad Prism Software 5.0 to calculate the half-maximal inhibitory concentration (IC₅₀).

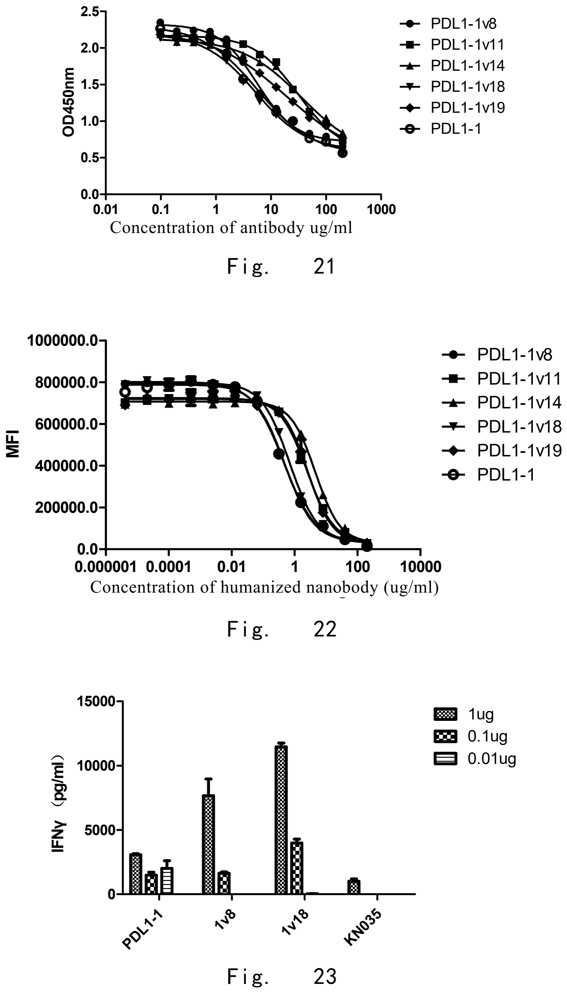

The assay results of blocking activities are shown in Figure 21. The competitive ELISA results (Table 19) indicate that the humanized antibodies PDL1-1v8 and PDL1-1v18 can block the binding of PDL1-his protein to PD1-Fc, demonstrating blocking activity similar to that of PDL1-1, with a dose-dependent relationship. The IC₅₀ values of PDL1-1v8 and PDL1-1v18 were 5.576 µg/mL and 5.216 µg/mL, respectively.

**Table 19**

| | PDL1-1v8 | PDL1-1v11 | PDL1-1v14 | PDL1-1v18 | PDL1-1v19 | PDL1-1 |
|---|---|---|---|---|---|---|
| IC₅₀ (µg/mL) | 5.576 | 31.13 | 37.06 | 5.216 | 18.8 | 5.805 |

### 14.5 Detection of the blocking activity of the humanized heavy-chain single-domain antibody PDL1-1 at the cellular level using FACS method.

Raji cells containing the full-length human PD-L1 gene plasmid were cultured to the logarithmic growth phase, resuspended in FACS buffer, adjusted to the cell density of 3×10⁶ cells/mL, and then plated at 100 µL per well. 50 µL/well of PD1-Fc and 50 µL/well of the test antibody were added. The cells were resuspended, and incubated at 4°C for 60 minutes. After been washed for three times with 200 µL/well of PBS, the cells were centrifuged at 300 g for 5 minutes to discard the supernatant. The secondary antibody SA-488 was diluted in FACS buffer, and the cells were resuspended at 100 µL/well, and incubated at 4°C for 60 minutes. The cells were washed for three times with 200 µL/well of PBS, and centrifuged at 300 g for 5 minutes to discard the supernatant. The cells were resuspended in 100 µL/well of PBS and subjected to detection on the instrument.

The results are shown in Figure 22 and Table 20. The humanized antibodies PDL1-1v8 and PDL1-1v18 can block the binding of Raji cells to PD1-Fc, exhibiting blocking activities similar to that of PDL1-1 in a dose-dependent manner. The IC₅₀ values of PDL1-1v8 and PDL1-1v18 were 0.458 µg/mL and 0.6938 µg/mL, respectively.

**Table 20**

| | PDL1-1v8 | PDL1-1v1 1 | PDL1-1v1 4 | PDL1-1v1 8 | PDL1-1v1 9 | PDL1-1 |
|---|---|---|---|---|---|---|
| IC₅₀ (µg/mL) | 0.458 | 2.477 | 4.585 | 0.6938 | 2.593 | 0.477 |

### 14.6 Activation effects of PD-L1 single-domain antibodies and humanized antibodies on PBMCs

Peripheral blood mononuclear cells (PBMCs) were isolated from the peripheral blood of two healthy donors using a human peripheral blood lymphocyte separation tube (Dayou) by density gradient centrifugation. Anti-CD3 antibody was diluted at 1/2000 and added to the cells, and PDL1-1, 1v8, 1v18, and KN035 were added at concentrations of 1 µg, 0.1 µg, 0.01 µg, and 0 µg, respectively. 1E5 PBMCs were added to each well, and cultured for 5 days. The level of IFN-γ in the supernatant was detected using an interferon-gamma (IFN-γ) detection kit.

The results are shown in Figures 23-24. The PDL1 single-domain antibody PDL1-1 and the humanized antibodies 1v8 and 1v18 can enhance the secretion of interferon-gamma (IFN-γ) by PBMCs, indicating that the PDL1 single-domain antibody promotes the activation of PBMCs in a dose-dependent manner.

### 14.7 Humanized PDL1-1 Nanobody enhances the killing of target cells by effector cells

Raji-PDL1 cells were revived at a concentration of 3E5/mL, and added at 50 µL per well. NK92 cells were added at an effector-to-target (E:T) ratio of 1:10 (the E:T ratio cytotoxicity assay is shown in the figure). The antibody was added at a concentration of 10 µg/mL, and incubated overnight for 20 hours. The obtained mixture was mixed, and CCK8 (from Absin) was added, and incubated at 37°C for 4 hours, and then the absorbance was measured at OD₄₅₀ wavelength.

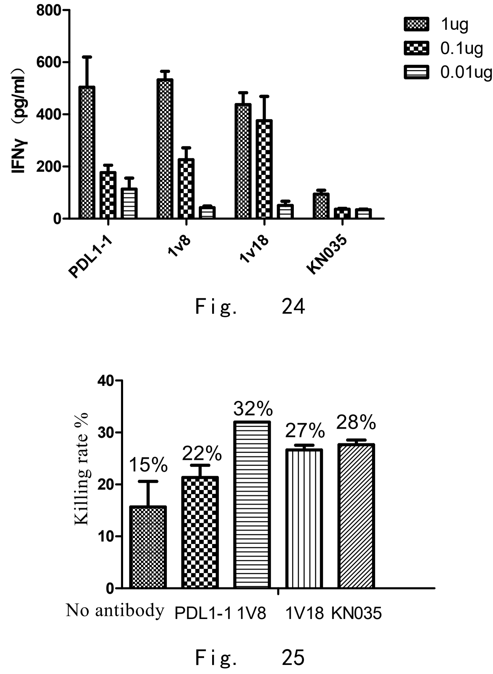

The results are shown in Figure 25. The killing effect of the humanized PDL1-1 heavy-chain single-domain antibody 1v8 against target cells was superior to that of KN035, while 1v18 exhibited comparable killing effect on KN035. However, both 1v8 and 1v18 were superior to the non-humanized antibody PDL1-1.

### Example 15

This example involves the fusion of a PD-L1-1 heavy chain single-domain antibody with murine IL-12.

### 15.1 Different formats of fusion proteins of the PDL1-1 heavy chain single-domain antibody with murine IL-12

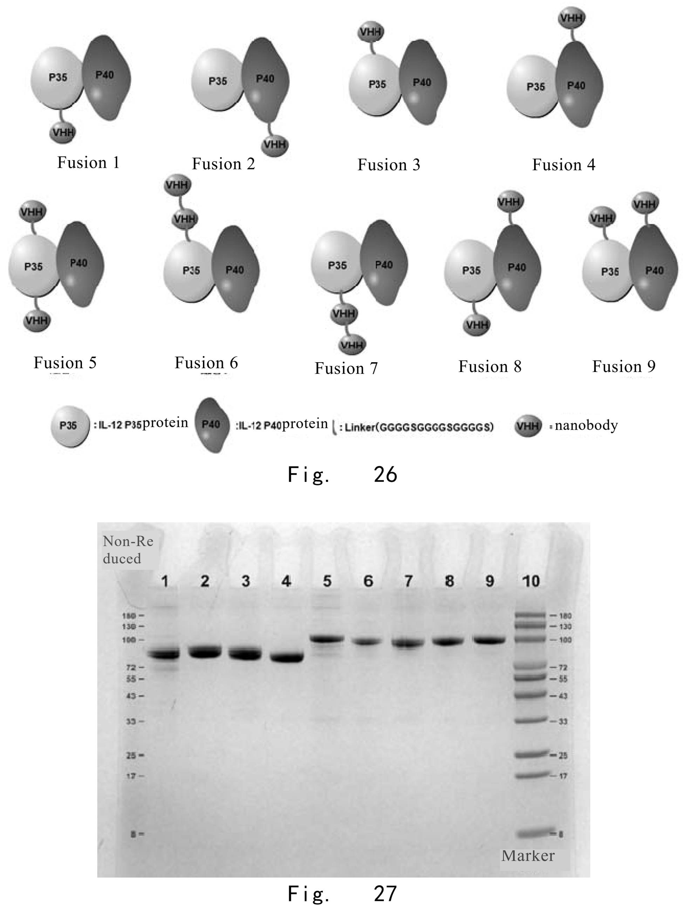

Different formats of fusion proteins of the PD-L1-1 heavy chain single-domain antibody with murine IL-12 were constructed. The murine P35/P40 and PD-L1-1 were linked using (4GS)3 to create 6 different ① to ⑥ formats respectively, as shown in Table 21. Plasmids were extracted, and co-transfected at a ratio of 1:1 to express the proteins. The simulated formats of the fusion proteins are illustrated in Figure 26. Among them, Fusion 1 to Fusion 4 refer to a single PD-L1-1 heavy chain single-domain antibody fused with murine IL-12, while Fusion 5 to Fusion 9 refer to two PD-L1-1 heavy chain single-domain antibodies fused with murine IL-12.

**Table 21**

| Fusion protein | Format |
|---|---|
| Fusion 1 | ① pXC17.4-mP35-P1-8his |
| | ② pCDNA3.1-mP40 |
| Fusion 2 | ③ pXC17.4-mP35-6his |
| | ④ pCDNA3.1-mP40- P1 |
| Fusion 3 | ⑤ pXC17.4-P1- mP35-8his |
| | ② pCDNA3.1-mP40 |
| Fusion 4 | ③ pXC17.4-mP35-6his |
| | ⑥ pCDNA3.1-P1-mP40 |
| Fusion 5 | ① pXC17.4-P1-mP35-P1-8his |
| | ② pCDNA3.1-mP40 |
| Fusion 6 | ③ pXC17.4- P1- P1-mP35-6his |
| | ④ pCDNA3.1-mP40 |
| Fusion 7 | ⑤ pXC17.4 - mP35-P1-P1-8his |
| | ② pCDNA3.1-mP40 |
| Fusion 8 | ① pXC17.4-mP35-P1-8his |
| | ⑥ pCDNA3.1-P1-mP40 |
| Fusion 9 | ⑤ pXC17.4-P1- mP35-8his |
| | ⑥ pCDNA3.1-P1-mP40 |

The fusion proteins were identified using non-reduced SDS-PAGE gel electrophoresis. The results are shown in Figure 27, wherein: 1: Fusion 1, 2: Fusion 2, 3: Fusion 3, 4: Fusion 4, 5: Fusion 5, 6: Fusion 6, 7: Fusion 7, 8: Fusion 8, 9: Fusion 9, 10: Protein molecular weight marker. The results indicate that the electrophoretic positions of the fusion proteins are consistent with expectations, the bands are clear, and the purity is high, with no broken or degraded bands observed.

### 15.2 PD-L1-binding ability of the PD-L1-1 heavy chain single-domain antibody fused with IL-12 protein

The human PDL1-Fc recombinant protein was coated on the plate at 200 ng/well and incubated overnight at 4°C. Subsequently, the PD-L1-1 humanized heavy chain single-domain antibody fused with IL-12 protein obtained from 15.1 was subjected to a serial 3-fold dilution across 12 gradients starting from 50 µg/mL, and the PD-L1-1 heavy chain single-domain antibody was subjected to a serial 3-fold dilution across 12 gradients starting from 10 µg/mL. The plate was blocked at 37°C for 1 hour, followed by incubation at 37°C for 1 hour for binding. The plate was washed, HRP-conjugated anti-His tag antibody (His Tag Antibody HRP, Sino Biological) was added, and the plate was incubated at 37°C for 45 minutes. The plate was washed, TMB substrate solution was added for color development, and the reaction was quenched with 1 M H₂SO₄. The absorbance was measured at 450 nm wavelength using a microplate reader. Data processing and parameter fitting were performed using GraphPad Prism Software 5.0 to calculate the EC₅₀ value.

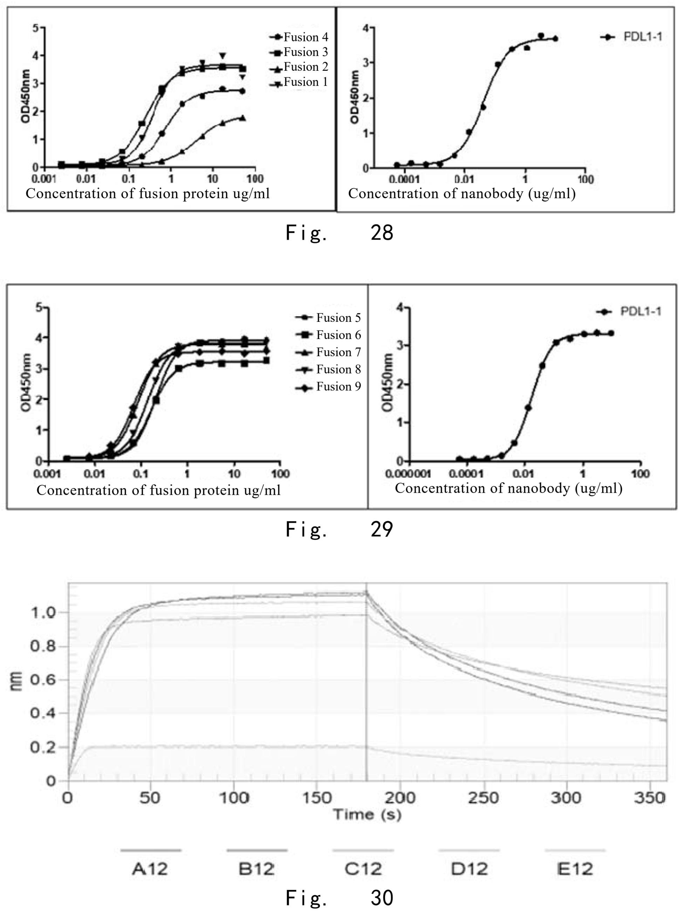

The results are shown in Figures 28-29, and Tables 22 and 23. The ELISA-based detection revealed that the binding activities of Fusion 2 and Fusion 4 proteins were relatively low, while those of Fusion 7 and Fusion 9 proteins were relatively high. The binding activities of the other fusion proteins were comparable.

**Table 22**

| | Fusion 1 | Fusion 2 | Fusion 3 | Fusion 4 |
|---|---|---|---|---|
| EC₅₀ (ug/ml) | 0.373 | 4.104 | 0.2514 | 0.7497 |
| EC₅₀ (nmol/L) | 4.93 | 54.29 | 3.33 | 9.91 |

**Table 23**

| | PDL1-1 |
|---|---|
| EC₅₀ (ug/ml) | 0.04208 |
| EC₅₀ (nmol/L) | 3.48 |

### 15.3 Determination of the change in the affinity of PD-L1-1 Heavy Chain Single-Domain Antibody fused with IL-12 Protein to PD-L1 by forteBIO^{™} System

ForteBIO^{™} Octet QK^{e} biomolecular interaction real-time detection system is an eight-channel detection platform based on Bio-Layer Interferometry (BLI). Based on the high-throughput characteristics of this platform, we conducted a comparative analysis of the kinetic profiles of interactions between different mutant antibody molecules and various mutant antigen molecules. The specific methods are as follows:
1) The parental PDL1-Fc and fusion proteins were diluted respectively to 100 nM in 10 mM PBS + 0.05% Tween-20 buffer, and added at 200 µL per well into a 96-well plate for detection;
2) The Loading > Baseline > Association > Dissociation procedure was conducted, the Association phase was set to 2 minutes and the Dissociation phase was set to 3 minutes. Both the Baseline and Dissociation processes are carried out in 10 mM PBS + 0.05% Tween-20 buffer, the Loading phase corresponded to a specific concentration of the PDL1-Fc dilution, and the Association phase corresponded to a specific concentration of the mutant dilution;
3) The results were fit and analyzed by Data Analysis to obtain the equilibrium dissociation constant K_{D} of the different fusion proteins as the ratio kdis/kon.

The results are shown in Figures 30-31 and Tables 24-25. According to the ForteBio assay, Fusion 9 exhibited an affinity of 0.0589 nM to the PDL1 protein with the lowest dissociation rate (kdis). Fusion 5-Fusion 8 showed comparable performance. Fusion 1 and Fusion 2 had relatively higher equilibrium dissociation constants (K_{D}), while Fusion 3 and Fusion 4 showed K_{D} values similar to that of PDL1-1.

**Table 24**

| | | KD (M) | kon(1/Ms) | kdis(1/s) |
|---|---|---|---|---|
| A12 | Fusion 1 | 1.35E-08 | 4.77E+05 | 6.46E-03 |
| B12 | Fusion 2 | 1.15E-08 | 6.39E+05 | 7.35E-03 |
| C12 | Fusion 3 | 4.05E-09 | 9.24E+05 | 3.75E-03 |
| D12 | Fusion 4 | 7.40E-09 | 6.47E+05 | 4.78E-03 |
| E12 | PDL1-1 | 3.39E-09 | 1.66E+06 | 5.63E-03 |

**Table 25**

| | | KD (M) | kon(1/Ms) | kdis(1/s) |
|---|---|---|---|---|
| A12 | Fusion 5 | 2.39E-10 | 6.31E+05 | 1.51E-04 |
| B12 | Fusion 6 | 1.31E-10 | 7.13E+05 | 9.36E-05 |
| C12 | Fusion 7 | 2.13E-10 | 7.48E+05 | 1.60E-04 |
| D12 | Fusion 8 | 1.58E-10 | 9.25E+05 | 1.46E-04 |
| E12 | Fusion 9 | 5.89E-11 | 9.69E+05 | 5.71E-05 |
| F12 | PDL1-1 | 2.96E-09 | 1.80E+06 | 5.32E-03 |

### 15.4 Activation effect of PD-L1-1 heavy chain single-domain antibody fused with IL-12 protein on NK-92 cells

100 µL of the PD-L1-1 heavy chain single-domain antibody fused with IL-12 protein and the WHO IL-12 standard working solution were taken respectively, and added to a 96-well plate containing the prepared NK-92 cells, so that final titers of 500.00 U/mL, 166.67 U/mL, 55.56 U/mL, 18.52 U/mL, 6.18 U/mL, 2.06 U/mL, 0.685 U/mL, and 0.23 U/mL were obtained, respectively. Each concentration was tested in duplicate. The final cell concentration was 1×10⁵ cells/mL. The cells were stimulated for (24±2) hours, and the 96-well plate was centrifuged at 1000 rpm for 5 minutes. The supernatant was diluted at 60-fold using the standard diluent (1X Dilution Buffer R) from the ELISA kit. 5 µL of the supernatant was taken and added to 295 µL of the standard diluent. The plate was shaken on a plate washer at high speed for 5 minutes. 50 µL of 1X Biotinylated Antibody was added to each well, shaken on a plate washer for 30 seconds to mix homogeneously, and incubated at room temperature for 120 minutes. The plate was washed, and 100 µL of 1X Streptavidin-HRP was added to each well and incubated at room temperature for 20 minutes. The plate was washed, and 100 µL of TMB was added and incubated at 25°C in darkness for 18 minutes. 100 µL of Stop Solution was added to quench the reaction. The OD value at 450 nm was measured using a SpectraMax M2 microplate reader. The data were processed using GraphPad Prism 6 software, with the OD value as the y-axis and the dilution factor as the x-axis. The data were fit using a four-parameter logistic model to obtain the activity curve, Top value, Bottom value, and R² value, wherein the R² value is ≥ 0.95.

**Table 26**

| Lot No. | Sample titer (10^6U/ml) | Protein content (mg/ml) | Molar concentration (uM) | Specific activity of the sample (10^6U/uM) | Decrease-fold |
|---|---|---|---|---|---|
| Fusion 1 | 1.185796384 | 1.245 | 16.46825397 | 0.072004985 | 0.2367967 |
| Fusion 2 | 2.270666032 | 1.49 | 19.70899471 | 0.115209632 | 0.1479958 |
| Fusion 3 | 4.62416746 | 0.4587 | 6.067460317 | 0.762125703 | 0.0223724 |
| mIL-12 | 0.3054196 | 1.0658 | 17.91260504 | 0.017050541 | 1 |
| R202112001 | 11.91817317 | 1 | | | |
| Fusion 4 | 1.17833531 | 1.276 | 16.87830688 | 0.0698136 | 0.25009045 |
| Fusion 5 | 5.003232601 | 0.596 | 6.711711712 | 0.745448079 | 0.0234218 |
| Fusion 7 | 1.357483993 | 0.917 | 10.32657658 | 0.131455375 | 0.1328186 |
| mIL-12 | 0.312748962 | 1.0658 | 17.91260504 | 0.017459714 | 1 |
| R202112001 | 16.83726459 | 1 | | | |
| Fusion 6 | 9.546284306 | 1.43 | 16.1036036 | 0.592804228 | 0.0227643 |
| Fusion 8 | 0.374900535 | 1.03 | 11.5990991 | 0.032321522 | 0.4175158 |
| Fusion 9 | 1.367544038 | 0.917 | 10.32657658 | 0.132429564 | 0.1019013 |
| mIL-12 | 0.242678608 | 1.07 | 17.98319328 | 0.013494745 | 1 |
| R202112001 | 14.89835083 | 1 | | | |
| PDL1-1 | / | 0.9 | | / | / |
| R202112001 | 11.57800636 | 1 | | | |

As shown in Table 26, under the condition of equal molar mass, the activation effect of murine IL12 on NK-92 cells is set as 1. The activation effect of the fusion protein Fusion 4 on NK-92 cells was reduced by 2.5 times, while the other fusion proteins showed varying degrees of enhancement. Among them, the nanobody PDL1-1 in the fusion protein did not activate NK-92 cells. This indicates that there is a synergistic effect between murine IL12 and the heavy-chain single-domain antibody PDL1-1 in the fusion protein, thereby promoting the activation of NK-92 cells.

### 15.5 PD-L1-1 heavy chain single-domain antibody fused with IL-12 enhances the killing effects of effector cells against target cells.

MDAMB231 cells were plated at a density of 3E5/mL, 50 µL per well. NK cells were added at effector-to-target (E:T) ratios of 1:2.5 and 1:5, with the antibody at a concentration of 5 µg/mL, and incubated overnight for 20 hours. The obtained mixture was mixed well and CCK-8 (from Absin) was added, and incubated at 37°C for 4 hours. The absorbance was measured at OD₄₅₀ nm to calculate the killing rate.

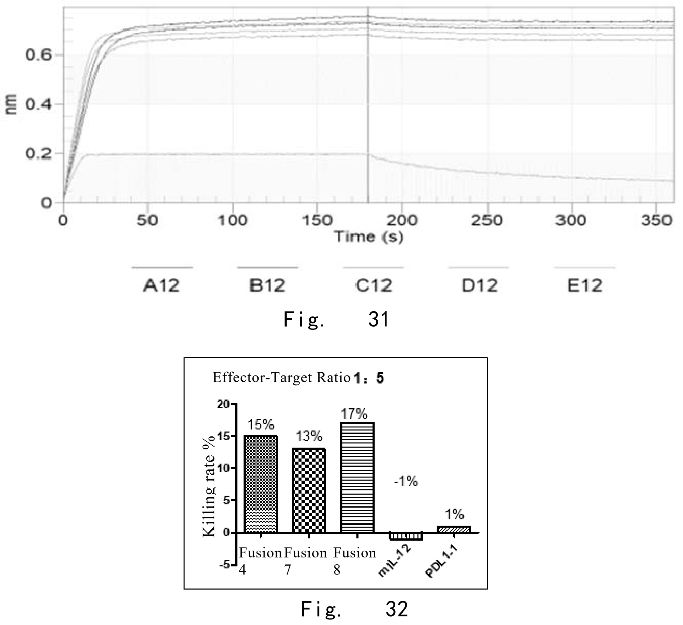

As shown in Figure 32, all of the fusion proteins PD-L1-1 heavy chain single-domain antibody fused with IL-12s(fusion 4, fusion 7, and fusion 8) induced greater killing effects on target cells than murine IL-12 alone or the PD-L1-1 heavy chain single-domain antibody alone, which demonstrates the synergistic effect between murine IL-12 and the PD-L1-1 heavy chain single-domain antibody in the fusion proteins.

In summary, the present invention has screened and prepared an anti-PD-L1 nanobody. This anti-PD-L1 nanobody exhibits high affinity and specificity, can efficiently target PD-L1, has a simple structure, is easily produced, and can be further developed into PD-L1-binding molecules, immunoconjugates, and the like, and thus holds broad application prospects in fields such as immunoassays and tumor therapy.

The applicant declares that the present invention is described in detail through the embodiments mentioned above. However, the present invention are not intended to limit the scope of the present invention in any way. A skilled person will understand that any improvements to the present invention, equivalent substitutions of the various raw materials of the products of the invention, additions of auxiliary components, and selections of specific methods shall fall within the scope of protection and the scope of disclosure of the present invention.

## Claims

1. An anti-PD-L1 nanobody, wherein the complementarity-determining regions of the VHH chain of the anti-PD-L1 nanobody are selected from any one of following groups:
(1) CDR1, CDR2, and CDR3 with amino acid sequences respectively shown in SEQ ID NO: 1, SEQ ID NO: 2, and SEQ ID NO: 3;
(2) CDR1, CDR2, and CDR3 with amino acid sequences respectively shown in SEQ ID NO: 4, SEQ ID NO: 5, and SEQ ID NO: 6;
(3) CDR1, CDR2, and CDR3 with amino acid sequences respectively shown in SEQ ID NO: 7, SEQ ID NO: 8, and SEQ ID NO: 9;
(4) CDR1, CDR2, and CDR3 with amino acid sequences respectively shown in SEQ ID NO: 10, SEQ ID NO: 11, and SEQ ID NO: 12.

2. The anti-PD-L1 nanobody of claim 1, wherein the amino acid sequence of the VHH chain of the anti-PD-L1 nanobody is selected from any one of the sequences shown in SEQ ID NO: 13 to SEQ ID NO: 16 or a sequence having at least 80% sequence identity thereto.

3. A PD-L1 binding molecule, comprising the anti-PD-L1 nanobody or the heavy chain variable region VHH of claim 1 or 2.

4. The PD-L1 binding molecule of claim 3, wherein the PD-L1 binding molecule is a monomer, dimer, or multimer.

5. A humanized antibody or an active fragment thereof, which is derived from the nanobody of claim 1 or 2.

6. The humanized antibody of claim 5, wherein the humanized antibody has the amino acid sequence shown in any one of SEQ ID NOs: 23-42; preferably, the amino acid sequence shown in any one of SEQ ID NOs: 30, 33, 36, 40, and 41; more preferably, the amino acid sequence shown in SEQ ID NO: 30 or 40.

7. A chimeric antigen receptor (CAR), wherein the CAR comprises an extracellular domain, and the extracellular domain comprises an anti-PD-L1 nanobody of claim 1 or 2, or a humanized antibody of claim 5 or 6.

8. A fusion protein, which comprises:
(Z1) a first protein, wherein the first protein includes: an anti-PL-L1 nanobody of claim 1 or 2, an anti-PD-L1 binding molecule of claim 3 or 4, or a humanized antibody or an active fragment thereof of claim 5 or 6;
(Z2) a second protein, wherein the second protein includes an Fc fragment or a cytokine; and
(Z3) an optional linker between the first protein and the second protein.

9. The fusion protein of claim 8, wherein the interleukin is IL-12; or the Fc fragment is an Fc fragment of human IgG.

10. A nucleic acid molecule, which comprises an encoding nucleic acid of the anti-PD-L1 nanobody of claim 1 or 2, the anti-PD-L1 binding molecule of claim 3 or 4, the humanized antibody or an active fragment thereof of claim 5 or 6, the chimeric antigen receptor of claim 7, or the fusion protein of claim 8 or 9.

11. A recombinant expression vector, which comprises the nucleic acid molecule of claim 10.

12. A recombinant cell, which comprises the nucleic acid molecule of claim 10 or the recombinant expression vector of claim 11.

13. A method for preparing the anti-PD-L1 nanobody of claim 1 or 2, which comprises:
inserting a gene encoding the anti-PD-L1 nanobody of claim 1 or 2 into an expression vector to obtain a recombinant vector; introducing the recombinant vector into a host cell for culture; and isolating and obtaining the anti-PD-L1 nanobody from the culture.

14. Use of the anti-PD-L1 nanobody of claim 1 or 2 in targeted binding to PD-L1.

15. Use of the anti-PD-L1 nanobody of claim 1 or 2, the anti-PD-L1 binding molecule of claim 3 or 4, the humanized antibody or an active fragment thereof of claim 5 or 6, the chimeric antigen receptor of claim 7, the fusion protein of claim 8 or 9, the nucleic acid molecule of claim 10, the recombinant expression vector of claim 11, or the recombinant cell of claim 12, in the preparation of a medicament for the targeted therapy of a tumor;
preferably, the tumor includes a PD-L1-expressing tumor;
preferably, the PD-L1-expressing tumor includes any one of gastric cancer, lung cancer, liver cancer, osteosarcoma, breast cancer, pancreatic cancer, lymphoma, ovarian cancer, esophageal cancer, bladder (urothelial) cancer, melanoma, Merkel cell carcinoma.

16. An immunoconjugate, which comprises the anti-PD-L1 nanobody of claim 1 or 2, the anti-PD-L1 binding molecule of claim 3 or 4, the humanized antibody or an active fragment thereof of claim 5 or 6, the chimeric antigen receptor of claim 7, the fusion protein of claim 8 or 9 and a conjugation moiety;
and the conjugation moiety is selected from any one or a combination of at least two of a protein, a small molecule compound, a fluorophore, a radioisotope, a contrast agent, or a fatty acid.

17. A pharmaceutical composition, comprising any one or a combination of at least two of the following: the anti-PD-L1 nanobody of claim 1 or 2; the anti-PD-L1 binding molecule of claim 3 or 4; the humanized antibody or an active fragment thereof of claim 5 or 6; the chimeric antigen receptor of claim 7; the fusion protein of claim 8 or 9; the nucleic acid molecule of claim 10; the recombinant expression vector of claim 11; the recombinant cell of claim 12; or the immunoconjugate of claim 16.

18. An engineered immune cell, which comprise the recombinant expression vector of claim 11, or has an exogenous nucleic acid molecule of claim 10 integrated into the genome thereof, or expresses the chimeric antigen receptor of claim 7.

19. A kit, which comprises:
(1) a first container, wherein the first container contains the anti-PD-L1 nanobody of claim 1 or 2, the anti-PD-L1 binding molecule of claim 3 or 4, the humanized antibody or an active fragment thereof of claim 5 or 6, the chimeric antigen receptor of claim 7, the fusion protein of claim 8 or 9, the recombinant cell of claim 12, or the immunoconjugate of claim 16, or a combination thereof; and/or
(2) a second container, wherein the second container contains a secondary antibody against the contents of the first container;
or
the kit comprises a detection plate, which comprises: a substrate (support plate) and a test strip, and the test strip comprises the anti-PD-L1 nanobody of claim 1 or 2, the anti-PD-L1 binding molecule of claim 3 or 4, the humanized antibody or an active fragment thereof of claim 5 or 6, the chimeric antigen receptor of claim 7, the fusion protein of claim 8 or 9, the recombinant cell of claim 12, or the immunoconjugate of claim 16, or a combination thereof.

20. A method for preventing and/or treating a PD-L1-related disease, the method comprising administering to a subject in need thereof the anti-PD-L1 nanobody of claim 1 or 2, the anti-PD-L1 binding molecule of claim 3 or 4, the humanized antibody or an active fragment thereof of claim 5 or 6, the chimeric antigen receptor of claim 7, the fusion protein of claim 8 or 9, an immunoconjugate of claim 16; a pharmaceutical composition of claim 17, or an engineered immune cell of claim 18, or a combination thereof.

21. A diagnostic method for PD-L1-related diseases, comprising steps of:
(i) obtaining a sample from a subject to be diagnosed, and contacting the sample with an anti-PD-L1 nanobody of claim 1 or 2, an anti-PD-L1 binding molecule of claim 3 or 4, a humanized antibody or an active fragment thereof of claim 5 or 6, an immunoconjugate of claim 16, or a combination thereof; and
(ii) detecting whether an antigen-antibody complex is formed, wherein the formation of the complex indicates that the subject is a confirmed patient with PD-L1-related diseases.
